# EUROPEAN PATENT SPECIFICATION

(11) **EP 2 729 531 B1**
(45) Date of publication and mention of the grant of the patent: **19.09.2018**
(21) Application number: 12778047.6
(22) Date of filing: 03.07.2012
(51) Int. Cl.: C09D 153/00, C09D 5/02, C09D 5/16, C08F 293/00

(54) **FORMULATION**
FORMULIERUNG
FORMULATION

(30) Priority: 04.07.2011 GB 201111439
(43) Date of publication of application: 14.05.2014
(73) Proprietor: Syngenta Limited, Bracknell, Berkshire RG42 6EY (GB)
(72) Inventor: MULQUEEN, Patrick Joseph, Bracknell Berkshire RG42 6EY (GB); THOMSON, Niall Rae, Bracknell Berkshire RG42 6EY (GB); BIGGS, Simon Richard, Brisbane QLD 4072 (AU); CHAGNEUX, Nelly, F-LS2 9DF Leeds (FR); DUBOIS, Mathieu Edmond René, 7180 Seneffe (BE); SARKER, Prodip, Cambridge CB4 0PE (GB); SCANLON, Shane, Halifax, West Yorkshire HX4 8 NH (GB)
(74) Representative: Syngenta International AG
(86) International application number: PCT/EP2012/062943
(87) International publication number: WO 2013/004704

(56) References cited:
- EP-A1- 1 760 527
- EP-A1- 2 729 002
- EP-A2- 0 067 479
- WO-A1-96/00567
- WO-A1-2010/038046
- WO-A2-2011/028996
- US-A- 4 735 015
- US-A1- 2010 103 524
- US-A1- 2010 323 884
- DAVID S. GERMACK ET AL: "Influence of the structure of nanoscopic building blocks on the assembly of micropatterned surfaces", JOURNAL OF POLYMER SCIENCE PART A: POLYMER CHEMISTRY, vol. 44, no. 17, 1 September 2006 (2006-09-01), pages 5218-5228, XP055045077, ISSN: 0887-624X, DOI: 10.1002/pola.21614
- LIXIN SONG ET AL: "One-step synthesis of titania nanoparticles from PS-P4VP diblock copolymer solution", NANOTECHNOLOGY, IOP, BRISTOL, GB, vol. 18, no. 13, 4 April 2007 (2007-04-04) , page 135605, XP020118960, ISSN: 0957-4484, DOI: 10.1088/0957-4484/18/13/135605

## Description

The present disclosure relates to the use of AB diblock copolymer micelles dispersed in an apolar liquid medium as hydrophobic surface treatments. Also disclosed is the novel use of AB diblock copolymer micelles dispersed in an apolar liquid medium and the use of compositions thereof which self assemble into aggregate structures in a suitable medium, and to a method suitable for preparing a surface treatment using the same which provides functional benefits associated with hydrophobic surface treatments such as oil and/or water repellence, anti-ice and dirt-repellence properties. The disclosure is equally applicable to both large surfaces and discrete objects. In addition, the treatment of surfaces with the AB diblock copolymers according to the present disclosure can yield supplementary benefits such as oleophobic, anti-bacterial or anti-fungal properties. The present invention relates to a process for coating an object with micelles which comprise an AB block copolymer comprising the step of treating the object with an apolar liquid containing the micelles.

### Background to the Invention

The controlled wetting of surfaces has many potential applications such as the waterproofing of surfaces, fabrics, concrete, paints, windows and windshields. In addition, controlled solid-liquid interfacial properties can have benefits in producing low friction surfaces for use in areas such as swimsuits, diving gear, boats and ships, as well as micro-fluidic devices. Additionally, small objects such as seeds and organic crystals can benefit from such waterproofing/protective surface treatments. Controlled wetting can also have implications for controlling/preventing the build-up of ice, for example on aircrafts and refrigeration equipment.

Further applications in the area of "easy-to-clean" surfaces/coatings are also possible {Emma Dorey, Chemistry& industry, issue 18, 5 (September 2006); Ralf Blossey, Nature Materials, vol. 2, 301-306, (2003)}. Such surfaces are usually designed to facilitate cleaning by minimising the adhesion of dirt and promoting water repellence such that as water "rolls off' the surface it collects the poorly adhered dirt particles {Jon Evans, Chemistry& industry, issue 18, 16-17 (September 2006)}. Often, "self-cleaning surfaces" such as those described in WO 96/04123 are termed "Lotus-effect" surfaces or coatings, and the technology is termed "Lotus-Effect" technology. Such "self-cleaning surfaces" can be produced in different ways: by creating the surface structures directly from hydrophobic polymers during manufacture or by creating the surface structures after manufacture (specifically by imprinting or etching, or by the adhesion of a polymer made of hydrophobic polymers to the surfaces).

A variety of methods for controlling the wetting of surfaces have been reported, {Mathilde Callies, David Quéré, Soft matter, vol 1, 55-61, (2005); Taolei Sun, Wenlong Song, Lei Jiang, Chem.Comm., 1723-1725, (2005)} based on both the control of the surface chemistry and the surface morphology {S. Herminghaus, Europhys. Lett., 52, 165, (2000); J. Bico, U. Thiele, D. Quéré, Colloids Surf., A, 206, 41, (2002); H. Li, X. Wang, Y. Song, Y. Liu, Q. Li, L. Jiang, D. Zhu, Angew. Chem. Int. Ed., 40, 1743,(2001); L. Feng, S. Li, H. Li, J. Zhai, Y. Song, L. Jiang, D. Zhu, Angew. Chem. Int. Ed., 41, 1221, (2002); L. Feng, Y. Song, J. Zhai, B. Liu, J. Xu, L. Jiang, D. Zhu, Angew. Chem. Int. Ed., 42, 800, (2003); T. Onda, S. Shibuichi, N. Satoh, K. Tsujii, Langmuir, 12, 2125, (1996)}. More recently, combinations of these two approaches have been used {Jon Evans, Chemistry& industry, issue 18, 16-17 (September 2006); Igor Luzinov, Sergiy Minky, Vladimir V.Tsukruk, Prog.Polym.Sci., vol 29, 635-698, (2004)}. It is known for example from basic surface wetting theory that a low energy surface (with a concomitant large contact angle, greater than 100°) will tend to repel water. The result will be the formation of drops that roll off the surface easily.

US2002/0048679 (and related EP 1018531A1) describe surfaces from which water runs off easily as having to be either very hydrophilic or hydrophobic. Hydrophilic surfaces have low contact angles with water, and this brings about rapid distribution of the water on the surface and finally rapid run-off of the resultant film of water from the surface. In contrast, hydrophobic surfaces form droplets through large contact angles with water. These droplets can roll off rapidly from inclined surfaces.

Many materials are known to be capable of producing water repellence. In general the materials possess a very low dielectric constant and are uncharged organics. Amongst these are materials such as halogenated organic polymers, for example polytetrafluoroethylene (PTFE) and derivatives thereof {Anthony M.Granville, William J.Brittain, Macromol. Rapid.Comm., vol 25, 1298-1302, (2004; Lei Thai, Fevzi C.Cebeci, Robert E.Cohen, Michael F.Rubner, NanoLetters, vol 4, 7, 1349-1353, (2004; Motoshi Yamanaka, Kazuki Sada, Mikiji Miyata, Knji Hanabusa, Kazunori Nakano, Chem Comm, 2248-2250, (2006)}. One approach for manufacturing such surfaces is to apply a thin layer of a new material with the appropriate characteristics (for example appearance, durability, adhesion and application requirements) directly onto the surface of interest. Such surface coatings or surface treatments should be easily and uniformly applied; established within a reasonable amount of time and process constraints; have a minimal environmental impact with respect to their synthesis and application; resist the effects of environmental assault; and provide good economic value.

The main problems with such materials to date include;
(a) Determining the best method to deposit the materials onto a surface of interest since the materials are often soluble in a limited number of organic/volatile solvents. One possibility is for example a spin-casting method. However, this method usually requires the liberal use of solvents with the associated cost and environmental concerns.
(b) The durability of the coatings when applied and used in 'real applications' is an issue. Damage of the coatings through abrasion and the impact of harsh external conditions can compromise their efficiency. For example, re-coating can not only be difficult but also expensive and is still subject to the same environmental concerns.
(c) Photodegradation effects caused by sunlight can also compromise the surface integrity and lead to re-application needs.
(d) Application of such treatments can be costly and requires complex and difficult to scale procedures during the manufacturing process.
(e) The coating of small objects (nanometre to millimetre sized), especially effective coating of small particles such as organic crystals with a polymer is difficult to achieve. Many techniques have been applied to coat particles, such as those based on Wurster coating technologies for spray dry coatings wherein a fluidised bed of dry particles has a coating solution sprayed onto the fluidised bed and solidified on the particles by either evaporation of a volatile solvent in the coating solution or cooling to set the coating polymer (if applied in molten form). Such techniques are notoriously variable, in that it is difficult to avoid agglomeration of the particles into larger masses and the coating can be extremely ineffective in coating all surfaces and edges of a particle (especially a small crystalline particle with variable edges and sides of crystal). This limitation can be partially overcome by employing higher quantities of coating composition but this can significantly alter the properties of the organic particle being coated as well as having an impact on the economics of the process and product cost. Coating of particles in a liquid medium is highly attractive if a technique could be identified. Work with dispersions of organic pesticides in water (WO2006/015791) in the presence of reactive monomers produced "coated" particles as dispersions in water but these are matrix particles where the particle is engulfed in a polymer during the polymerisation process. Many similar techniques produce such matrix particles.

Random fluorinated copolymers prepared by radical copolymerisation of monomers in solution in a water-miscible organic solvent using peroxides or azo compounds as initiators have been described, together with their hydrophobic and oleophobic properties on various substrates (see, for example, EP542598, US1106630 and US2004026053).

US5,324,566 describes the use of hydrophobic fluorinated siloxane polymers for producing water repellent surfaces and it discloses that the water repelling properties of the fluorinated siloxane material can be improved by forming surface irregularities on the surface of such a material. It is for example mentioned that the surface is modified with irregularities of a height from about 0.1 micrometer up to the wavelength of visible light. Likewise, US5,599,489 and EP0933388A2 describe how the structured surface includes fluorine containing polymers or has been treated using alkylfluorosilanes.

US2002/0048679 describes surfaces having a smooth, extremely hydrophobic polymer film (for example, polytetrafluoroethylene) and surfaces having a smooth extremely hydrophilic polymer film as examples where water and dirt run off without forming droplets. US2002/0048679 further describes how a 'long-term' hydrophobic coating may be formed by applying certain silane derivatives underneath a hydrophobic coating on a surface. Other self-cleaning surfaces are described in US2002/0150723, US2002/0150724, US2002/0150725, US2002/0150726, US2003/0013795 and US2003/0147932.

US3,354,022 discloses water repellent surfaces having a rough micro structure with elevations and depressions and a hydrophobic material based on a fluorine containing polymer. According to one embodiment, a surface with a self-cleaning effect can be applied to ceramic, brick or glass by coating the substrate with a suspension comprising of glass beads (diameter of 3 to 12 micrometres) and a fluorocarbon wax which is a fluoroalkyl ethoxymethacrylate polymer. Unfortunately, such coatings have a disadvantage in that they possess a low abrasion resistance and only a moderate self-cleaning effect.

Further developments of surface coatings that are designed to produce strongly hydrophobic surfaces include the use of copolymers, polymer blends and mixtures of polymers and nanoparticles (such as titanium dioxide, as described in US6800354, US7112621B2, US7196043 and DE10016485.4). For example, coated surfaces have been produced using fluorocarbon polymers that can give contact angles of up to 120°. Titanium dioxide (TiO2) has also been used with such fluorinated surfaces. It is known that under UV irradiation the TiO₂ is photocatalytically active and can produce super-wetting properties as a result of water hydrolysis effects {Akira Nakajima, Kazuhito Hashimoto, and Toshiya Watanabe, Langmuir, 16 (17), 7044 -7047, (2000)}. However the addition of TiO₂ present with (fluoroalkyl)silane does not affect the hydrophobicity of the overall material; that is, the modified (fluoroalkyl)silane remains hydrophobic.

The preparation of such surfaces using nanoparticles suffers from several drawbacks including the use of organic solvents (US3354022) and the use of a subsequent heat treatment (US6800354). Thus, there is a need for a simple for producing surfaces that are "easy-to-clean" with water and are optically transparent.

It has also been demonstrated recently that the control over surface wetting can be improved by producing surfaces with a well-controlled micron-sized roughness {Eiji Hosono, Shinobu Fujihara, Itaru Honma, Haoshen Zhou; JACS, vol 127, 13458-13459, (2005); Xi Yu, Zhiqiang Wang, Yugui Jiang, Feng Shi, Xi Zhang, Adv. Mater. Vol 17, 1289-1293, (2005); A. A. Abramzon, Khimia i Zhizu (1982), no. 11, 38 40}. These rough surface features assist in producing 'ultrahydrophobic' substrates by physical methods that include trapping air and reducing contact areas between the water drops and the surface. The basic underlying surface should itself be hydrophobic and when combined with the roughness effects, it results in surfaces with contact angles greater than 150° which are extremely hydrophobic. However, such surfaces tend to be difficult to manufacture, they are usually very fragile and easily damaged and the micron-scale features can cause diffraction effects with light, which can be therefore problematic for use in applications involving glass.

Whilst many commercial surface coatings based on solutions of polymers in organic solvents are produced by drop-casting or spin coating, alternatives that are based on chemical grafting of polymer films have recently been discussed. Using this approach, coatings that comprise of dense brush-like films of polymers which are chemically attached to a surface are produced. The polymers detailed herein can have controlled chemistry that produces the desired wettability characteristics. Furthermore, the inherent chemical variety available to the synthetic polymer chemist means that such layers can be produced with a wide variety of physical properties, as well as the opportunity for including a stimulus responsive surface.

Stimuli-responsive polymers {J.Rodriguez-Hernandez, F.Checot, Y.Gnanou, S.Lecommandoux, Prog.Polym.Sci., 30, 691-724, (2005)} are polymers that are able to respond to small changes in their environment with a corresponding large change in a specific physical property. Typical stimuli include: temperature, pH, ionic strength, light-, electric- and magnetic fields. Some polymers respond to a combination of two or more of these stimuli. For coatings or surface treatments, stimulus responsive polymers have the potential to be used in a wide variety of applications where controlled changes in properties such as adhesion, lubrication and wetting are required.

WO08071957 and WO10038046 describe the novel composition and/or novel use of AB block copolymers comprising both fluorinated and non-fluorinated portions that can form micelle structures and can be employed to surface coat structures such as fabrics, concrete structures, glass windscreens, glass structures to render then "stay-clean" by a combination of dust repellence and water sheeting effects. However, these compounds were used solely in a water based formulation or mainly organic polar solvents to produce hydrophilic surface treatments, as they offer water sheeting properties.

It has been demonstrated that although these surface treatments give some easy clean characteristics, they lack other important functions such as water repellence and anti-ice that may be beneficial on a wide variety of substrates. Hence, there is a need for an alternative surface treatment which is as convenient and demonstrates such easy clean properties.

Surfactants are recognised to adsorb uniquely to interfaces such as oil/water interfaces and solid/liquid interfaces and are employed as stabilisers to produce dispersions of particles in a liquid medium (such as water) that remain stable to agglomeration on storage. Because of this property of adsorbing as a monomolecular layer at an interface, surfactants in the form of polyelectrolytes have been employed to produce layers of surfactant on a substrate such as a solid particle. Such processes (for example, as in WO2000/077281) are slow to build-up oppositely charged single layers of polyelectrolyte (each layer being only the thickness of a surfactant monolayer and many layers being required to build up a coating thickness of utility). Surfactants can also aggregate into structures containing many surfactant molecules in a single aggregate. These aggregates are called micelles. They are commonly spherical in shape but can have a large range of shapes and structures. The number of molecules that compose such an aggregate can be very many, often in the order of hundreds of molecules. Micelles can be composed of relatively simple surfactant structures but can also be composed of high molecular weight block-copolymer surfactants. Moreover, even large complex block copolymers can form micelles. Such block copolymer micelles, when comprised of oppositely charged micelles, have been induced to adsorb in a layer by layer (LbL) manner onto spherical colloidal particles to produce coatings on particles such as a latex or a spherical silica particle (NSTI-Nanotech 2007, www.nsti.org, ISBN 1420061836 Vol. 2, 2007 pp13-16 *and* Adv. Mater. 2007, 19, 247-250).

We have now unexpectedly and surprisingly found that the use of complex copolymer micelles dispersed in an apolar liquid medium as coating agents for agrochemical organic crystals produces surface coatings with high loadings of copolymer in a single treatment (or very few treatments) and such products find utility in a range of applications, particularly but not limited to the agrochemical field.

In one aspect, the present disclosure provides an apolar liquid comprising micelles which themselves comprise a copolymer and in which the micellar cores are more hydrophilic than the micelle coronas.

In another aspect, the present disclosure provides a surface coated with micelles which comprise a copolymer; where the micellar cores are more hydrophilic than the micellar coronas.

In one aspect, the present invention provides a process for coating an agrochemical organic crystal comprising the step of treating the agrochemical organic crystal with an apolar liquid in which micelles are dispersed.

Examples of such small materials are objects that need to be protected from their environment, for example water soluble organic crystals that may be otherwise incompatible in an aqueous formulation or particles which may react with the other ingredients of the formulation causing an increase of viscosity and decrease in the shelf life of the formulation. Other objects may be seeds, plant tissue such as foliage, leaves, flowers or seed heads, organic and inorganic crystals, solid particles (TiO₂, CaCO₃, SiO₂, gold, latex particles etc). Objects that are of irregular shape and size are especially well suited to be coated by this technology as the micelles coat all edges and corners of objects uniformly although regular shapes such as spherical particles (latex particles, spherical silica for example) are equally well coated.

Products of utility may be an agrochemical, laundry chemicals, cosmetics, food additives, paint and coating additives, biocides for paints, pharmaceutical or other particles that find utility in various fields. The novel coating, produced by micelle-forming polymers, finds utility in a variety of ways. The coated particle can now be more effectively targeted for adhesion to a substrate by selection of the block copolymer, as in targeting a specific substrate in agriculture such as an insect cuticle, leaf surface or fungal pathogen or in pharma, for delivery to a specific target organ or protection of an agent for delivery through the mammalian stomach for selective and protected delivery later in the digestion system or in laundry for release of an agent at the appropriate point in the wash cycle. Moreover, the effectively coated particles confer greater colloidal stability on systems, allowing greater and improved stability when mixed with other components.

### Further suitable applications include, without limitation:

Sustained release or controlled release usages, for example: pharma, for example acid resistant structures (oral delivery past low pH in the stomach), protection of labile actives, pseudo-zero order release through the micelle layer and Ostwald-ripening resistant formulations; cosmetics; perfumes, for example slowing down evaporation of top-notes or sustained release and minimising overpowering odours; particles having affinity for cellulose and trapped on textile surface during laundering; flavours, for example light-stabilised to prevent oxidation; self-healing coatings, for example particle induced to burst to release a resin that repairs damage; carbonless copy paper; novel, double taste and texture food, for example a particle which dissolves in the mouth and releases a new taste; pressure sensitive adhesives; sealants; nutrition (for example increased bioavailability of complex molecules and protection of sensitive molecules such as vitamins, probiotics and other food additives); toner inks with photosensitivity or thermal sensitivity; textile coatings, for example, for altering permeability properties; antifouling coatings; surface protective coatings, for example, for improving scratch or abrasion resistance; and construction materials, for example wall-boards, plasterboards and cements.

It is well known that chemical incompatibility between different components in liquid laundry formulations can cause instability in these formulations. In particular, laundry chemicals including bleaching chemicals and bleach activation agents such as, but not limited to, sodium percarbonate and tetraacetylethylenediamine (TAED) that are widely used in powder laundry formulations are incompatible with liquid laundry detergents. Bleach activation agents, precursors and catalysts tend to be unstable in many liquid formulations and although the surfactants in the liquid formulation are stable they can react with bleach or bleach activator chemicals or catalysts or derivatives of them. One solution is to add a solid form bleach activator as a separate dose to the liquid laundry detergent, but this is inconvenient for the consumer. The present disclosure provides a means of protecting the solid bleach activator form interaction with water and other liquid detergent components to enable a stable liquid detergent to be formulated.

It is desirable to be able to control pH during a laundry cycle and to this end it would be advantageous to be able to release sodium carbonate into the laundry medium at a given point in the cycle in response to increased dilution with water. Sodium carbonate coated with reverse micelles as described in this disclosure is an efficient means of achieving this.

The sustained release of biocides and anti-fouling agents is of commercial interest to the paints and coatings industry and in particular for marine applications. One example of a biocide that has been employed as an antifouling agent for marine use is DCOIT (4,5-dichloro-2-n-octyl-3(2H)-isothiazolone). This active has low solubility in sea water which is extremely desirable, however in solvents used in paint formulations such as xylene, it is extremely soluble. This means that it is likely to react with the paint binders within the formulation and may increase the paint viscosity or induce plasticizing of the paint.

Marine paint manufacturers will benefit from a biocide that improves in-can stability of the paint whilst incorporating sustained release of the active after application onto the marine vessel. The present disclosure provides a means of protecting the biocide from the other active ingredients in a paint formulation and provides a means of sustained release in sea water.

Safe delivery of active pharmaceutical ingredients (APIs) to the intended target site within a mammalian body is a major area of both commercial unmet need and scientific research. In many cases the API needs to be protected from interaction with its environment in order to prevent unwanted chemical reaction or biological use of the active at the wrong site within the body or at the wrong rate. One solution to this problem is to formulate the API into a tablet and to add a protective or enteric coating to the tablet. This can be sub-optimal for a number of reasons including patient preference for non-tablet formulation and the potential risk of over-dosing [if the enteric coating fails]. The present disclosure enables individual crystals of API to be coated enabling formulation into a capsule rather than a tablet and minimizing the risk of over-dosing [as the coating would need to fail multiple times on individually coated API crystals rather than only once on the tablet].

Non Steroidal Anti Inflammatory Drugs (NSAIDs) such as ibuprofen and diclofenac are limited in their administration because at higher doses, side effects (such as gastric erosion, thrombasthenia or thrombocytopenia, and fluid retention) may become severe.

Taste masking of APIs is a current target for the pharmaceutical industry as numerous actives in the preparations are bitter or undesirable in taste. Applying a polymer coating to the API is one approach to achieving taste masking by providing an inert coating to prevent its dissolution. Successful taste masking can be evaluated by determining the rate of release of the active from the coated particles.

Vitamin C is also known as ascorbic acid, ascorbate and ascorbate monoanion. It is the enolic form of an α-ketolactone. Vitamin C works physiologically as a water soluble antioxidant by virtue of its high reducing power. It acts as singlet oxygen quenchers, and it is capable of regenerating vitamin E. Vitamin C is called an antioxidant because of its ability of quenching or stabilizing free radicals that lead over time to degenerative diseases, including cancer, cardiovascular disease and cataracts.

Ascorbic acid properties are impaired by its high reactivity, and hence, poor stability in solution, which can result in heavy losses during food processing. It can be degraded rapidly in the presence of oxygen in free-radical mediated oxidative processes. The processes are strongly catalysed by transition metal ions, especially iron and cooper, leading to rapid destruction of the ascorbate. Oxidation is also accelerated at neutral pH and above.

Destruction can occur in the presence of enzymes such as ascorbate oxidase and ascorbate peroxidase.

The food industry may employ microencapsulation to produce foods which are more nutritionally complete. The properties of microencapsulated nutrients will allow the food processor greater flexibility and control in developing foods with high nutritional value. Ascorbic acid is added extensively to many types of food products for two quite different purposes: as a vitamin supplement to reinforce dietary intake of vitamin C, and as an antioxidant, to protect the sensory and nutritive quality of the food itself.

The present disclosure enables individual crystals of ascorbic acid or other food supplements to be coated for application in the food industry as fortification. Coated particles could be potentially incorporated in dry form into cake mixes, puddings, gelatine desserts, chewing gum, milk powder, jellies, pet foods, breakfast cereals, in short, into products with low water activity.

It is therefore an object of the present disclosure to provide compositions that can be deposited easily onto a substrate surface without the need for expensive processing and has versatility that it can be applied equally well on large flat surfaces as well as contiguously to coat objects such as seeds, plant material, inorganic or organic particles (including polymer particles) and crystalline material such as inorganic or organic crystals.

A polymer or polymeric composition prepared according to the present disclosure may be coated onto a preferred substrate by any established coating process, for example, but not limited to for example a spray process. Methods of exposing the substrate to the solution include for example any known technique for forming a coating from a solution such as spin coating, dip coating, roller coating, brush coating, curtain flow or spraying, roller coating, wire-bar coating, extrusion coating, air knife coating, curtain coating and slide coating. More preferably dipping and spraying ensures that every part of the surface has been wetted by the treatment composition. The treatment can be applied to both interior and exterior surfaces.

Various surfaces may be treated including for example metals, metal alloys, glasses, plastics, textiles, rubber, porcelain, ceramics, tile, enamelled appliances, polymers (for example polyurethanes, polyesters, polyacrylics and polycarbonates), resins (for example melamine/phenolic resins), painted surfaces, natural surfaces (like wood) and cellulose substrates.
1) The metal or metal alloy object or articles may comprise a metal or metal alloy selected from the group comprising: aluminum, magnesium, beryllium, iron, zinc, stainless steel, nickel, nickel-cobalt, chromium, titanium, tantalum, rare earth metal, silver, gold, platinum, tungsten, vanadium, copper, brass and bronze; and combinations or derivatives thereof; and plated articles thereof.
2) The plastic objects or articles may comprise a polymer selected from the group comprising: transparent or non-transparent polyurethane, polycarbonate, polyethers, polyesters such as polyethylene terephthalate, polyvinyl chloride, polystyrene, polyethylene, polyvinyl acetate, silicone rubbers, rubber latex, polycarbonate, cellulose esters polycarbonate, polyester-polyether copolymers, ethylene methacrylates, polyolefins, silicone, natural and synthetic rubbers, nylon and polyamide; and combinations thereof.
3) The glass objects or articles may comprise, at least partially, a material selected from the group comprising: glass, such as optical glasses, optical lenses, polarizing glasses, mirrors, optical mirrors, prisms, quartz glass and ceramics; and combinations thereof.

The substrate may include an exterior surface or article member, such as for example: a window sash, structural member or windowpane of a building; an exterior member or coating of a vehicle such as automobile, railway vehicle, aircraft and watercraft; an exterior member, dust cover or coating of a machine, apparatus or article; and an exterior member or coating of a traffic sign, various display devices and advertisement towers, that are made, for example, of metal, plastics or glass or a combination thereof.

Examples of substrates, include, but are not limited to: medical devices, protection shields, window sheets, windowpane, greenhouse walls, freezer doors, food packaging foils and printing paper.
1) The metal objects can include for example: freezer doors, mirrors, condenser pipes, ship hulls, underwater vehicles, underwater projectiles, airplanes and wind turbine blades.
2) The plastic objects can include for example: face shields, helmet shields, swim goggles, surgeon face shields, food packaging, plastic foil, greenhouse walls, greenhouse roofs, mirrors, wind shields, underwater moving objects, airplane windows and shields.
3) The glass objects can include for example: window glasses, greenhouse, glasses, glass sheets, face shields, optical glasses, optical, lenses, polarizing glasses, mirrors, optical mirrors, prisms, quartz glass, parabolic antennas, automobile head beam light glasses, automobile windshields, airplane control light glasses, solar panels and solar concentrator mirrors and runway lights.

The coating may also be applied to clear plastic or glass used for example as protective shields, windows, windshields, greenhouse panels, food packaging foils, goggles, optical glasses and contact lenses.

Likewise the coating may be applied for example: to an exterior surface of a telescope lens, especially a riflescope, a spotting scope, or a binocular to reduce the likelihood of fogging or distortion due to the collection of moisture on the lens without significantly reducing light transmission through the lens in the visible range. That is, scopes used by sportsmen, the military and the like.

Exterior or interior parts of a building may also benefit from the coating for example: windowpanes, toilets, baths, wash basins, lighting fixtures, kitchenware, tableware, sinks, cooking ranges, kitchen hoods and ventilation fans, which are made from metal, glass, ceramics, plastics, a combination thereof, a laminate thereof or other materials.

Also disclosed is a novel surface treatment that promotes variable wetting properties on the surface, or in other words provides an "easy-to-clean" surface, meaning that an identifiable cleaning benefit ("easier-to-clean", "cleaner-longer", "stay-clean" etc.). Examples include micelles applied to surfaces of chemical reactors to make them easy to clean; micelles applied to the inside surfaces of pipes and tubes to make them easy to clean; micelles applied to the surfaces of road vehicles, trains and aeroplanes to make them easy to clean; and micelles applied to the surfaces of food packaging materials to prevent food build up on the packaging.

Also disclosed is the provision of novel compositions that are able to demonstrate a hydrophobic effect which is desirable for different application areas, such as water repellence, anti-ice and water barrier properties for small objects.

Also disclosed is the provision of novel compositions that are able to vary or reverse the water repellent properties when the local environmental conditions are changed (such as temperature, salt concentration or pH).

The surface treatment disclosed is hydrophobic. The properties and associated benefits are achieved using simple processing and application techniques.

The present process is according to claim 1.

The AB block copolymer comprises two blocks (A and B) which have different affinities for a liquid medium such that micelles form in the liquid medium.

Although the micelles are formed in a liquid medium, any eventual coated particles may be present not only in a liquid composition but alternatively in a dry, solid composition [for instance, due to an evaporation step or a drying step].

A preferred AB block copolymer comprises:
(i) a first hydrophobic block A, comprising a polymer selected from the group consisting of a homopolymer of an acrylate or alkylacrylate (preferably an acrylate or C₁₋₄ alkylacrylate; more preferably an acrylate or methacrylate) monomer; a copolymer comprising two or three different monomers selected from acrylate or alkylacrylate (preferably an acrylate or C₁₋₄ alkylacrylate; more preferably an acrylate or methacrylate) monomers; a homopolymer of a styrenic derivative monomer; a copolymer comprising two different monomers selected from styrenic derivative monomers; a homopolymer of an alkene or diene monomer; a copolymer comprising two different monomers selected from alkene and diene monomers; a homopolymer of a heterocyclic monomer; a copolymer comprising two different monomers selected from heterocyclic monomers; and a random, alternating, gradient or block copolymer comprising monomers selected from acrylate monomers, alkylacrylate (preferably C₁₋₄ alkylacrylate; more preferably methacrylate) monomers, styrenic derivative monomers, alkene monomers, diene monomers and heterocyclic monomers; and
(ii) either a second hydrophobic block B or a hydrophilic block B having a different affinity than the block A for the liquid medium in which the AB copolymers are dispersed such that micelles are formed.

Throughout the disclosure, references to alkyl and alkylene groups and moieties, relate to both straight-chained and branched versions.

Preferably any acrylate or alkylacrylate monomer is, independently, of formula A' 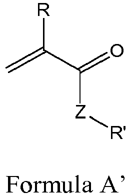 wherein R is H or a C₁ to C₄ alkyl chain; Z is O, a phosphorous derivative [preferably PH₃] or a nitrogen derivative [preferably NH]; R' is selected from the group comprising: C₁ to C₁₈ alkyl; alkylaminoalkylene containing from 1 to 18 carbon atoms (preferably from 2 to 18 carbon atoms); alkoxyalkylene containing from 1 to 18 carbon atoms (preferably from 2 to 18 carbon atoms); C₁ to C₁₈ dihydroxyalkyl; C₁ to C₁₈ silylalkyl; C₁ to C₁₈ epoxy alkyl; phosphoryl; phosphoryl C₁ to C₁₈ alkyl; a vinyl phosphonate or phosphoric acid monomer; and a methacrylate having at least one crosslinkable function or one UV or thermal-responsive unit; where each alkyl or alkylene group is, independently, fluorinated or non-fluorinated.

Preferably any styrenic derivative monomer is, independently, of formula B' 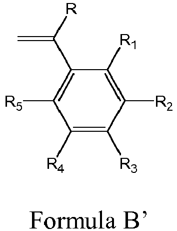 wherein R is H or a C₁ to C₄ alkyl group; and R₁, R₂, R₃, R₄ and R₅ are each independently H or a C₁ to C₈ alkyl group or a halogen atom [preferably chlorine or fluorine].

Preferably any alkene or diene monomer is, independently, of formula Cₐ or C_{b} 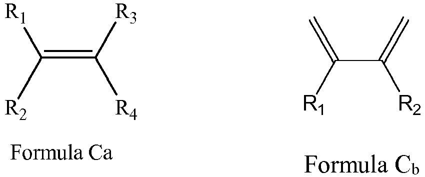 wherein R₁, R₂, R₃ and R₄ are each independently selected from H and C₁ to C₄ alkyl (preferably R₁, R₃ and R₄ are each H; and R₂ is H or C₁ to C₄ alkyl).

Preferably any heterocyclic monomer is, independently, of formula Dₐ, D_{b}, D_{c} or D_{d} 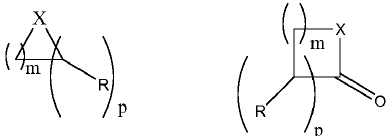 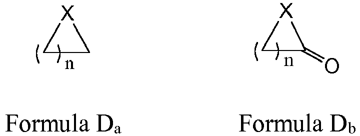 wherein n is from 1 to 7, m is from 0 to 5 and p is from 1 to 7; R is H or a C₁ to C8 alkyl group; and X is O, N or S.

The ratio of the monomers in each block of block copolymer AB is such that the weight fraction of the (hydrophobic) block A and the (hydrophobic or hydrophilic) block B agents leads to the formation of organised aggregates, such as micelles. The number of the monomers comprising the block copolymer AB is: preferably from 5 to 250 units of A; more preferably from 10 to 200 units of A; and most preferably from 15 to 150 units of A; and, likewise, preferably from 5 to 250 units of B; more preferably from 10 to 200 units of B; and most preferably from 15 to 150 units of B.

A suitable alkylacrylic or acrylate monomer of Formula A' is when Z is O; and R' is a C₁ to C₁₈ alkyl group (more preferably a C₁ to C₈ alkyl group); another suitable monomer of Formula A' is provided by Formula 1: 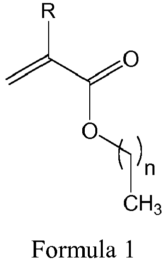 where n is 1 to 17, more preferably 1 to 8.

A suitable fluorinated alkylacrylic or acrylate monomer of Formula A' is when Z is O; and R' is a fluorinated alkyl group; another suitable monomer of Formula A' is provided by Formula 2: 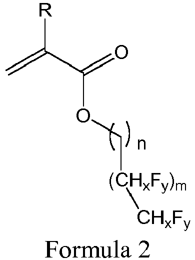 where n is 1 to 6 and the chain is linear or non-linear, more preferably 1 or 2; m is 0 to 7 and the chain is linear or non-linear, x is 0 to 2 and y is 3-x.

A suitable alkylacrylic or acrylate monomer for Formula A' is when Z is O; and R' is an alkylaminoalkyl group containing up to eighteen carbon atoms. Another suitable monomer of Formula A' is provided by Formula 3: 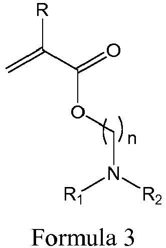 where R₁ and R₂ are each independently H, a C₁ to C₆ alkyl group; phenyl; benzyl or cyclohexyl; and n is from 1 to 17; more preferably, R₁ and R₂ are each methyl and n is from 1 to 5.

A suitable alkylacrylic or acrylate monomer for Formula A' is when Z is O; and R' is an hydroxyalkyl containing up to 18 carbon atoms. Another suitable monomer of Formula A' is provided by Formula 4a or 4b: 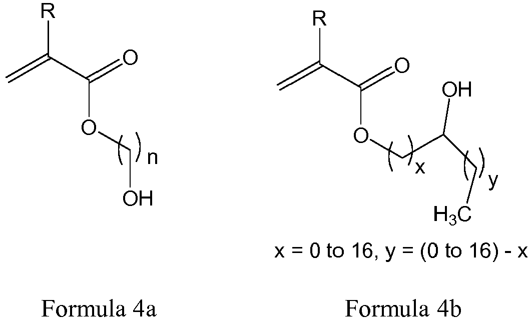 where n is 1 to 18 and the chain is linear or non-linear (more preferably n is from 1 to 4) and x and y are each 0 to 16, more preferably 0 to 6. Suitably, for Formula 4b, x = 0 to 16; y = 0 to 16; and x + y ≤ 16.

A suitable alkylacrylic or acrylate monomer for Formula A' is when Z is O; and R' comprises a dihydroxyalkyl group. Another suitable monomer of Formula A' is provided by Formula 5a or 5b: 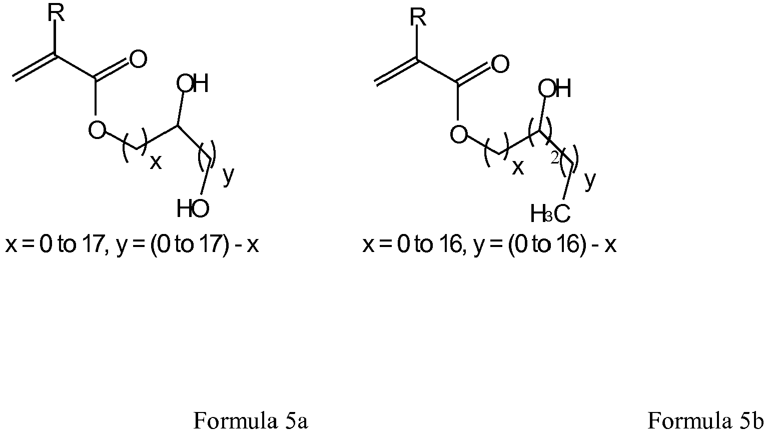 where x and y are each 0 to 17 in Formula 5a or 0 to 16 in Formula 5b; more preferably x and y are each 0 to 7 in Formula 5a or 0 to 6 in Formula 5b (and the chain can be linear or non-linear). Suitably, for Formula 5a, x = 0 to 17; y = 0 to 17; and x + y ≤ 17. Suitably, for Formula 5b, x = 0 to 16; y = 0 to 16; and x + y ≤ 16.

A suitable alkylacrylic or acrylate monomer for Formula A' is when Z is O; and R' is a C₁ to C₁₇ silylalkyl group. Another suitable monomer of Formula A' is provided by Formula 6a or 6b: 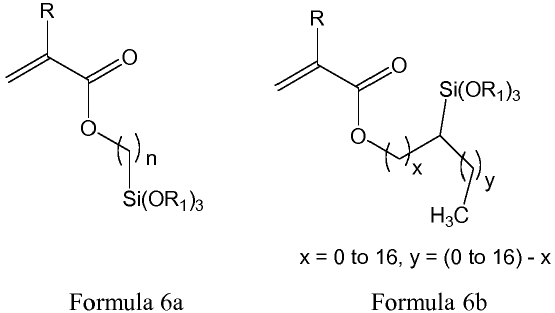 where R₁ is H or C₁ to C₄ alkyl and x and y are each from 0 to 16, preferably from 1 to 6. Suitably, for Formula 6b, x = 0 to 16; y = 0 to 16; and x + y ≤ 16.

A suitable alkylacrylic or acrylate monomer for Formula A' is when Z is O; and R' is an epoxy alkyl group. Another suitable monomer of Formula A' is provided by Formula 7a or 7b: 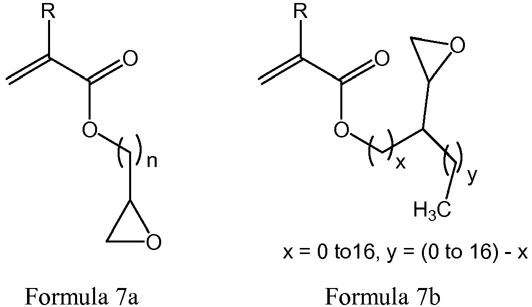 where x and y are each from 0 to 16, preferably from 0 to 6. Suitably, for Formula 7b, x = 0 to 16; y = 0 to 16; and x + y ≤ 16.

A suitable monomer of Formula A' is when Z is O; and R' is a phosphoryl or phosphoryl alkyl group. Another suitable monomer of Formula A' is provided by Formula 8a or 8b: 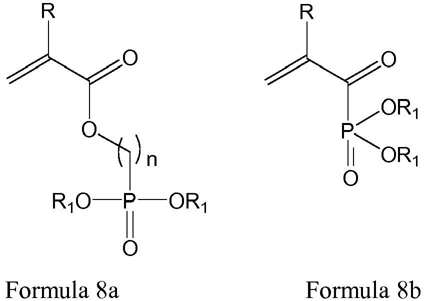 where each R₁ is independently H or C₁ to C₆ alkyl, preferably H or methyl.

Suitable monomers of Formula B' are independently selected from styrene, α-methylstyrene, 2-methylstyrene, 4-methylstyrene, 2,4-dimethylstyrene, 2,4,6-trimethylstyrene, 4-isopropylstyrene, 2-fluorostyrene, 3-fluorostyrene, 4-fluorostyrene, 2,6-difluorostyrene, 2,3,4,5,6-pentafluorostyrene, 2-chlorostyrene, 3-chlorostyrene, 4-chlorostyrene and 2,6-dichlorostyrene and other vinyl substituted aromatics.

Suitable monomers of Formula Cₐ or C_{b} are independently selected from ethylene, propylene, butylene, butadiene and isoprene.

Suitable monomers of Formula Dₐ or D_{b} or D_{c} or D_{d} are independently selected from ethylene oxide, propylene oxide, butylene oxide and caprolactone type monomers (such as ε-caprolactone or γ-butyrolactone, lactide, oxiran-2-one, 1,3-dioxolane and caprolactam).

When the Block B is hydrophobic, it may comprise one or more monomers, independently selected from the monomers defined above. Block B is chosen to have a different affinity to the liquid medium to Block A. The structures outlined for Block A can be applied for Block B provided Block A and B are different to each other.

When the block B is hydrophilic, a number of chemicals may be employed for the hydrophilic component B, all of which need to be water-soluble; examples may be selected from the group comprising:
hydrophilic organic monomers, oligomers, prepolymers or copolymers derived from vinyl alcohol, N-vinylpyrrolidone, N-vinyl lactam, acrylamide, amide, styrenesulfonic acid, combinations of vinylbutyral and N-vinylpyrrolidone, methacrylic acid, acrylic acid, vinylmethyl ether, vinylpyridylium halide, melamine, maleic anhydride/methyl vinyl ether, vinylpyridine, ethyleneoxide, ethyleneoxide ethylene imine, glycol, vinyl acetate, vinyl acetate/crotonic acid, methyl cellulose, ethyl cellulose, carboxymethyl cellulose, hydroxyethyl cellulose, hydroxypropyl cellulose, hydroxymethyl ethyl cellulose, hydroxypropylmethyl cellulose, cellulose acetate, cellulose nitrate, hydroxyalkyl (alkyl)acrylate such as hydroxyethyl (meth)acrylate, hydroxypropyl (meth)acrylate, alkylaminoalkyl (alkyl)acrylate, 2-(dimethyl amino) ethyl methacrylate, 2-(diethyl amino) ethyl methacrylate, 2-(diisopropyl amino) ethyl methacrylate, 2-(N-morpholino)ethyl methacrylate, or a derivative thereof, ethylene glycol (meth)acrylates (for example triethylene glycol (meth)acrylate) and (meth)acrylamide), N-alkyl (meth) acrylamides (for example N-methyl (meth)acrylamide and N-hexyl (meth)acrylamide), N,N-dialkyl (meth)acrylamides (for example N,N-dimethyl (meth)acrylamide and poly-N,N-dipropyl (meth)acrylamide), N-hydroxyalkyl (meth)acrylamide polymers, such as poly-N-methylol (meth)acrylamide and poly-N-hydroxy ethyl (meth)acrylamide, and N,N-dihydroxyalkyl (meth)acrylamide polymers, such as poly-N,N-dihydroxyethyl (meth)acrylamide, ether polyols, polyethylene oxide, polypropylene oxide, and poly(vinyl ether), alkylvinyl sulfones, alkylvinylsulfone-acrylates, (alkyl)acrylate with a pendent phosphorus group such as vinylphosphonate, vinylphosphonic acid, vinylphosphine oxide and any (alkyl)acrylate with a ester function -COOR such as R is CₓH₂ₓPO₃R₂ wherein x is 2 to 10, most preferably x is 2, and R is a hydrogen or an alkyl group having 1 to 4 carbon atoms, preferably methyl; and related compounds or a combination thereof.

The polymers comprising the AB block copolymer comprise monomers, and the ratio of the monomers comprising each polymer of the block copolymer AB is such that the weight ratio of the (hydrophobic) block A to the (hydrophobic or hydrophilic) block B leads to the formation of organised aggregates. In addition, the weight fraction of the (hydrophobic) block A and the (hydrophobic or hydrophilic) block B leads to the formation of micelles. Certain copolymers disclosed have been found to form complex and large micelles in solution.
As stated above AB Block copolymers can comprise a hydrophobic ("water hating") block A and a second hydrophilic ("water loving") or two hydrophobic blocks A and B differentiated as having different solubility parameters for a same liquid medium. Variation in the copolymer properties can be obtained by varying the monomer types (different available chemistries), the molecular weights of the copolymer (at a fixed ratio of the two component block sizes) and the ratio of the molecular weights of the constituent blocks (at a fixed overall molecular weight for the copolymer).

Importantly, to form micelles (that is, aggregates formed by molecules of block coplymer) in an apolar liquid medium, the insoluble (or poorly soluble in the liquid medium) blocks drive the formation of aggregates of the molecules. The structures of the aggregates are dependent on the copolymer concentration and the exact nature of the copolymer molecules. Schematically, micelles may be seen, for example, as a spherical aggregate having two parts; one core composed of the copolymer block insoluble or less soluble in the liquid medium and one corona composed of a copolymer block having affinity for the liquid medium. Other micellar structures are possible and known to those skilled in the art.

The polymers comprising the AB block copolymer are comprised of monomers, and the ratio of the monomers comprising each polymer of the block copolymer AB is such that the weight ratio of the hydrophobic block A and the more hydrophilic block B leads to the formation of organised aggregates. In addition, the weight fraction of the hydrophobic block A and the more hydrophilic block B leads to the formation of micelles. The copolymers disclosed have been found to form complex and large micelles in organic solution.

Once the AB block copolymer comprises two hydrophobic blocks A and B differentiated as having different solubility parameters for a same liquid medium, such structures will form micelles with a corona composed of block A in a liquid medium where the blocks B are less soluble than the blocks A in the chosen liquid medium or micelles with a corona composed of block B in a liquid medium where the blocks A are less soluble than the blocks B in the chosen liquid medium.

The chemistry for the micellar aggregates should be such that the micelles will adsorb freely onto a wide variety of particle surfaces. Also, the composition may form micelles and the aggregate structures of the composition preferably have a maximum dimension [diameter in the case of spherical micelles] of from to 3 to 500nm, preferably from 3 to 300nm. The block-copolymer micelle structures most preferably have a maximum dimension [diameter in the case of spherical micelles] of from 10 to 100nm.

In one aspect, the micelles each comprise from 10 to 1000 copolymer molecules.

The AB block copolymer may take the form of: linear block copolymer (diblock, triblock or multiblock), miktoarm copolymer (star copolymer), ladder (H-shaped) copolymer, graft and comb (co)polymer; preferably a linear block copolymer.

Also, the distribution of component monomers within each copolymer block is in the form of homo, random, gradient, alternative, block, graft and comb (co)polymers, any type of copolymer structures which will lead to a segregation of copolymers in the liquid media as organised aggregates.

It is also preferred that the block copolymer is preferably selected from the group comprising: AB blocks, ABA blocks, ABC blocks copolymers.

In a preferred example, the polymers used in the composition are prepared by controlled living radical polymerisation reactions. Preferably, the block copolymers according to the one aspect of the present disclosure are prepared by means of controlled living radical polymerisation to obtain narrow molecular weight distribution copolymers. Suitable synthetic routes include but are not limited to: Reversible Addition - fragmentation chain transfer (RAFT), Group transfer polymerisation (GTP) and Atomic transfer radical polymerisation (ATRP), Activated regenerated by electron transfer (ARGET), nitroxide-mediated polymerization (NMP), ring-opening polymerization and ionic type of polymerization and combinations of techniques where appropriate.

In a one aspect of the invention, the micelles may be crosslinkable and are optionally crosslinked (before, during or after treating the surface with the apolar liquid containing the micelles) resulting in a more durable coating on the substrate. In the current disclosure, crosslinking can be described as the physical and/or chemical interaction between chains of the AB diblock copolymer. The crosslinking can take place either in the core of the micelles, in the corona of the micelles and/or between the coronas of two contiguous micelles and the crosslinking may or may not be reversible.

Chemical crosslinking requires the use of a molecule called a crosslinker or crosslinking reagent. Three preferred chemical crosslinking strategies are: (1) crosslinking with a multifunctional organic compound, for example via condensation or addition reactions such as carboxylic acids with amines, carboxylic acids with hydroxyls, hydroxyls with isocyanates etc; (2) ring-opening reactions such as epoxy groups with amines and (3) radical initiated crosslinking of vinyl or similar chemical functions such as those in divinyl benzene and/or di-methacrylates, which can be introduced to the AB di-block copolymers.

A multifunctional organic compound is defined as an organic compound containing two or more functional groups that may react with functional groups described for the AB di-block copolymers used in this disclosure to form crosslinks. The functional groups in the organic compound may be any that will react with functions described herein for the AB di-block copolymer including but not limited to: amine, hydroxyl, carbonyls such as ketones or aldehydes, carboxyls such as carboxylic acid, isocyanates and sulfhydryl.

Vinyl groups may be introduced to the AB di-block copolymer by using vinyl compounds that also contain a function that will react with functional groups described in this disclosure for the AB di-block copolymer. Examples of such chemistry include, but are not limited to, amine functionalised vinyl compounds such as amino alkyl methacrylates. Following the introduction of the vinyl chemistry, crosslinking is carried out by radical initiation via thermal or UV curing.

Chemical covalent crosslinks are stable mechanically and thermally, so once formed are difficult to break, whereas physical crosslinks are reversible and the physical crosslinking process may or may not require the use of crosslinking agents. Physical crosslinking occurs when there is the formation of a physical interaction between functional groups located either in the AB diblock copolymer alone or between functional groups located in the AB diblock copolymer and in the multifunctional crosslinker. Techniques include, but are not limited to, dehydrothermal treatment, plasma treatment, hydrogen bonding, ionic interactions and freeze thawing.

Crosslinking (physical and/or chemical) can bring many benefits including making the hydrophilic corona of micelles more hydrophobic and controlling the release rate of an active coated with crosslinked micelles.

The block copolymer disclosed comprises at least one block that adsorbs to a target surface. The composition may further comprise an adhesion promoter. An adhesion promoter will generally consist of a polyelectrolyte of opposite potential (charge) to the potential (charge) of the crystal; in the case the block copolymer micelles coat which is targeted is of similar relative potential to that of the crystal to be coated.

Also in a composition disclosed the apolar liquid medium may comprise an organic solvent or mixtures of solvent, or an organic solvent free from water, and wherein the block copolymer is preferably completely dissolved in the liquid medium. To especially but not exclusively encourage reverse micellisation the liquid medium employed will consist of two solvents, one a good solvent for the block-copolymer and a second, less effective, solvent which will cause separation of the block co-polymer from solution and the formation of micelles.

Preferred apolar solvents, are in accordance with claim 7. Any solvent used conventionally in agrochemical formulations may be suitable for use in the present disclosure. Preferred apolar solvents can be also selected from what are generally classified as oils, such as high molecular weight alkanes, for example paraffinic oil; such as Isopar V and Exxsol D140; alimentary oil such as olive oil, soy bean oil and castor oil and the like and combinations thereof. Conventional ester solvents are also suitable.

Preferably the ratio of the number of Block A units to the number of Block B units is from 1:0.1 to 1:10; more preferably from 1:0.2 to 1:5; even more preferably from 1:0.25 to 1:4; yet more preferably from 1:0.5 to 1:2.

When the composition of the present invention comprises a liquid, the ratio by weight of block copolymer to the liquid medium is preferably from 1 : 100,000 to 1 : 1 (according to claim 8) ; more preferably from 1 : 10,000 to 1 : 2; especially from 1 : 5,000 to 1 : 5; and most preferably from 1 : 5,000 to : 1 : 10.

It will also be appreciated by one skilled in the art that the composition according to present disclosure may preferably further comprise additional components or auxiliary agents selected from for example but not limited to dispersants, perfumes, biocides, and stabilisers, surfactants or wetting agents, emulsifiers, colouring agents, dyes, pigments, UV absorbers, radical scavenger, antioxidant, anti-corrosion agent, optical brightener, fluorescers, bleaches, bleach activators, bleach catalysts, non-activated enzymes, enzyme stabilizing systems, chelants, coating aid, metal catalyst, metal oxide catalyst, organometallic catalyst, filmforming promoter, hardener, linking accelerator, flow agent, leveling agent, defoaming agent, lubricant, matte particle, rheological modifier, thickener, conductive or non-conductive metal oxide particle, magnetic particle, anti-static agent, pH control agents, perfumes, preservative, biocide, pesticide, anti-fouling agent, algicide, bactericide, germicides, disinfectant, fungicide, bio-effecting agent, vitamin, drug, therapeutic agent or a combination thereof.

We have now found that these micelle structures can be conveniently employed to coat small particulate materials such as organic crystals. The chemistry of these applications is thereby incorporated herein. Moreover, the technique is easy to employ and permits high coating weights to be applied over all a surface (including corners and edges of crystals if present). The micelle structures of this disclosure can produce a coating thickness typically up to 50nm in a single pass treatment-very much higher than any other known technique-while maintaining complete stability and non-agglomeration of the coated particle. Multi-coats produce even higher coating weights and thicknesses.

The block copolymers of the disclosure form micellar aggregates typically 3-300nm in size. Aggregation number is controlled by the chemistry of the block copolymer in terms of absolute chemistry, charge, molecular weight and the solution conditions under which the micelle is formed. Typical aggregation numbers for such a block copolymer micelle can be of the order of 100 molecules. Typical molecular weights of a block-copolymer of the disclosure are 3 000 to 100 000 Daltons but are specified within the chemistry.

Block copolymer micelles can be simply employed by adding to a dispersion of a particle in a carrier liquid and allowing to equilibrate. Confirmation of coating can be obtained by SEM observation and quantitative data by analysis of a sample for total active material content (where an active material is coated). Other techniques to induce micelle formation (such as pH shift, temperature, solvent exchange or dilution) can all be suitably employed. As an alternative process, a drying technique to remove either a solvent or to induce a chemical change-such as loss of ammonia in a drying process- can be employed.

The block copolymer disclosed comprises at least one block that adsorbs to a target surface. The composition may further comprise an adhesion promoter (AP). An AP will generally consist of a polyelectrolyte of opposite potential (charge) to the potential (charge) of the surface (crystal); in this case the block copolymer micelles coat the AP modified surface (crystal). This allows copolymer micelles of similar potential (charge) to the surface (crystal) to be deposited on the surface (through a surface -AP-block copolymer arrangement).

The products disclosed comprise a surface (for example a particulate material) covered with a coating of block copolymer micelles (including, uniquely, edges and corners as well as faces). A key aspect of the present disclosure is the ability to provide good coverage [and protection] for sharp features such as edges and, particularly, corners of crystals.

As further embodiments, micelles comprise a core and a corona that are chemically different. This difference can be exploited for further benefits. The micelle core can be selectively loaded with a component that dissolves or can be dissolved by a suitable solvent in the core chemistry. For example, the application of a photostabiliser by such a technique (by incorporating the photostabiliser into the micelle core which is then coated onto the crystal surface), will improve the ability to stabilise sensitive chemistry against photolytic degradation. Soil mobility of particles of a pesticide can be similarly enhanced by coating a stable polymer micelle onto the crystal surface (in combination with added specific surfactants that can promoted improve soil mobility). In some situations, pesticides can induce a phytotoxic response (in cotton for example) due to too rapid a photolytic degradation. Coating crystals in this manner with a polymer micelle containing a photostabiliser could reduce the rate of degradation.

In a further aspect, the ability to coat such block copolymer micelle systems onto a substrate provides an elegant procedure to prepare mixed products by coating a polymer micelle containing a first active onto a crystal surface of a second active (with the option of further actives being in a dissolved or other dispersed state). Further, such coated polymer systems could then be applied to relevant surfaces, these to include seeds or surfaces outwith of Crop Protection uses that could require protection against attack such as wood or on surfaces where a long lasting barrier might be required (such as to prevent ingress of termites, ants or spiders or to prevent fungal growth in sensitive situations-eg fungicides in/on wallboards).

Furthermore, the coated crystalline particle may be a biologically active compound [for example, an agrochemical] whilst the micelle core may be loaded with a second biologically active compound [for example, an agrochemical]. Alternatively two or more different biologically active compounds [for example, agrochemicals] may be mixed together as coated particles according to the present disclosure in such a manner that the micellar coatings overcome any potential incompatibility problems [for example, physical or chemical incompatibility].

A composition according to the present disclosure may suitably be an agrochemical formulation; the agrochemical formulation may comprise an agrochemical active ingredient (such as a fungicide, herbicide, insecticide or plant growth regulator) or it may comprise an adjuvant which is used to enhance the bioperformance of an agrochemical [either in the same formulation as the adjuvant or to be applied from a separate formulation]. The composition can be in the form of a concentrate which is diluted or dispersed in a spray tank prior to use, although ready-to-use compositions can also be made. The final dilution is usually made with water, but can be made instead of, or in addition to, water, with, for example, liquid fertilisers, micronutrients, biological organisms, oil or solvents. The compositions may be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), dispersible concentrates (DC), suspension concentrates (SC), capsule suspensions (CS; in which case, the particle is a microcapsule) and seed treatment formulations. The agrochemical formulation may be used to control or combat a pest [examples of agricultural pests include unwanted plants (weeds), insects and fungi].

In one aspect of the present invention the cores of the micelles contain a chemical [which preferably may be a photoprotectant, a biologically active compound or an adjuvant].

Also disclosed is the ability to coat such block copolymer micelle systems onto a substrate provides an elegant procedure to prepare mixed products by coating a polymer micelle containing a first active onto a crystal surface of a second active (with the option of further actives being in a dissolved or other dispersed state). Further, such coated polymer systems could then be applied to relevant surfaces that could require protection against attack such as wood or on surfaces where a long lasting barrier might be required (such as to prevent ingress of termites, ants or spiders or to prevent fungal growth in sensitive situations-e.g. fungicides in/on wallboards).

Further, non-limiting crop protection applications include particle coating leading to: reduced antagonism by altering availability between two or more actives, triggered release potential- triggers can be pH, light, water, enzymes and alteration of release profile. These release rate alterations may be possible not only in the products of the disclosure but also when subsequently applied (for example to seeds- triggered release from seeds by coating technology-triggers can be pH, light, water, enzymes. The size range of particles to be coated can vary enormously. Where the particle is an organic crystal, the size range [largest dimension] can usefully be from 10nm to 500microns, preferably 500nm to 100 microns (although technical material greater than 500 microns could be also coated and employed in some utilities (such as pharma) in a pre-granulation stage to protect a material). When the crystal size is small, the micelle size chosen to coat the particle has to be even smaller. Where the particle is a granule (or a spray-agglomerated granule), the size can vary from about 50 microns to a few millimetres.

The present disclosure is illustrated by the following examples.

### Example 1: Preparation of Polymers and Block Copolymers

The surface treatment of the present disclosure is hydrophobic. The copolymers described in this example are AB block copolymers comprising a substantially hydrophobic block A, and a substantially hydrophobic or hydrophilic block B which has a different affinity for or solubility parameter within the liquid medium where the copolymers are dispersed compared to block A, such that micelles form in the liquid medium.

Block A can comprise one or more monomers, for example; styrene (S) and styrene derivatives, methacrylate and derivatives such as 2-ethyl hexyl methacrylate (EHMA), lauryl methacrylate (LMA), octadecyl methacrylate (ODMA), glycidyl methacrylate (GMA) and propylene oxide (PO). Those skilled in the art will appreciate the synthesis described in this example is not limited to the monomers listed here.

In the current example, the hydrophobic or hydrophilic block B is composed of methacrylic acid (MAA), 2-hydroxyethyl methacrylate (HEMA) or 2-ethyl hexyl methacrylate but those skilled in the art will understand that other monomers leading to a hydrophilic block can also be used.
The copolymers used herein were produced by Reversible Addition-Fragmentation Chain Transfer (RAFT) according to the protocol described in the patent applications WO08071957 and WO10038046 or by nitroxide mediated polymerisation (NMP) according to the protocol described in the Arkema patent application WO2007/057620A1. Therefore the block copolymers may be prepared by means of controlled living polymerisation techniques, such as group transfer polymerisation (GTP), atomic transfer radical polymerisation (ATRP), and activated regenerated by electron transfer (ARGET) or activated generated by electron transfer (AGET) that can synthesize well-defined homopolymers and block copolymers.
In addition to controlled radical polymerization, in the case of an heterocyclic monomer such as propylene oxide, ring-opening polymerisation techniques can be used. Examples of the composition of new prepared copolymers are given in Table 1.2.

### A) Use of RAFT to synthesise copolymers

In this example, in addition to structures described in WO08071957 and WO10038046, new copolymers structures were produced by RAFT polymerization using the RAFT agent, 2-cyanoisopropyl dithiobenzoate (CPDB). Whilst the current example prepares the block copolymer CPDB, those skilled in the art will appreciate that other RAFT agents may be used.

### RAFT synthesis of Poly(EHMA-block-MAA) copolymer : P(EHMA-b-MAA)

A series of poly[EHMAₓ-*b*-MAA_{y}] copolymers were prepared by RAFT polymerization using CPDB as chain transfer agent, azobisisobutyronitrile (AIBN) as initiator and propan-2-ol (IPA) as a solvent. The synthesis was a two step process: First, the hydrophobic block (EHMA) was synthesised, then the synthesis of the hydrophilic block (MAA) was initiated from the PEHMA homopolymer.

### a) Synthesis of the block A: PEHMA.

EHMA (15g, 75.7mmol, 60eq), CPDB (0.31g, 1.26mmol, eq), AIBN (0.10g, 0.63mmol, 0.5eq) and IPA (solvent, 6.82g, 114mmol) were added in a two necked flask containing a magnetic stirrer equipped with a cooling column. The mixture was degassed by nitrogen bubbling and heated at 90°C in a thermostatically controlled oil bath under a nitrogen atmosphere. The reaction was left under stirring for a minimum of 2hour30minutes (in this example 3h15min). A sample of the crude mixture was withdrawn and analysed by size exclusion chromatography (SEC - See Figure 1.3), and by Proton Nuclear Magentic Resonance (¹H NMR). A conversion of 98% was determined by ¹H NMR in CDCl₃, hence the resultant product was P(EHMA)X homopolymer where x = 59.

### b) Synthesis of block Bfrom block A

30 minutes before the end of the first synthesis, MAA (6.54g, 76.0mmol, 60eq), AIBN (0.10g, 0.64mmol, 0.5eq) and IPA (solvent, 45.39g, 757mmol) were added in another flask containing a magnetic stirrer. The mixture was degassed by nitrogen bubbling.

At the end of the first synthesis (in the current example 3h15min), the thermostatically controlled oil bath was removed to stop polymerisation. The mixture containing the second monomer was then transferred into the initial two necked flask via a cannula. This flask was heated again at 85°C in the thermostatically controlled oil bath (equipped with a cooling column) under nitrogen atmosphere to achieve the preparation of the second block of copolymer. After a minimum of 2h30min (in this example 2h35min), a sample of the crude mixture was withdrawn and analysed by ¹H NMR and SEC (Figure 1.3).

A conversion of 88% was measured by ¹H NMR in DMSO. The resultant product was determined as P(EHMAₓ-*b*-MAA_{y}) copolymer where x = 59 and y = 53.

Other P(EHMAₓ-*b*-MAA_{y}) polymers were prepared with x=68 and y=25 and with x=33 and y=21. The generic structure of the corresponding P(EHMAₓ-*b*-MAA_{y}) copolymers is given below.

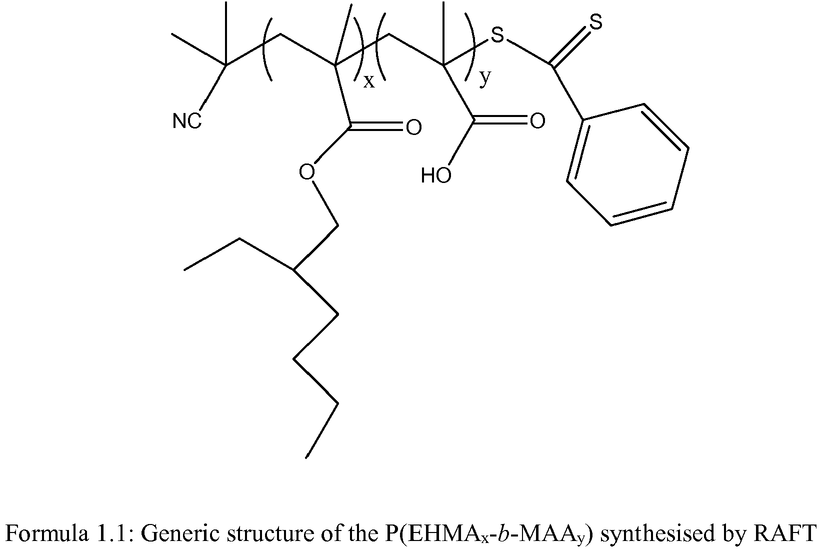

The P(EHMAₓ-*b*-MAA_{y}) copolymers can also be prepared by NMP, ATRP, GTP and indirect anionic polymerization.

### Preparation of other copolymers by RAFT synthesis.

Various block copolymers were synthesised. Block B was obtained from various methacrylated based monomers such as EHMA, LMA, ODMA and TFEMA. Block B was composed of hydrophilic units such as MAA and HEMA, or hydrophobic monomers such as EHMA. In this case, toluene was the solvent used for the synthesis instead of isopropanol.

The method described above for the synthesis of P(EHMAₓ-*b*-MAA_{y}) was used, which led for example to the successful synthesis of P(LMAₓ-b-EHMA_{y}) and P(ODMAₓ-b-MAA_{y}). The conversion rates, block sizes and reaction time are given in Table 1.2.

For the synthesis of (PEHMA₅₁-r-PGMA₂₂)-b-PMAA₄₇ the following protocol was used:

### a) Synthesis of the block A: PGMA and EHMA

GMA (3.29g, 23.2mmol, 25.6eq), EHMA (11.01g, 55.6mmol, 61eq), CPDB (0.22g, 0.9mmol, 1eq), AIBN (0.0784g, 0.5mmol, 0.5eq) and IPA (solvent, 24.32g, 406.5mmol) were added in a two necked flask containing a magnetic stirrer equipped with a cooling column. The mixture was degassed by nitrogen bubbling and heated at 82°C for 5 hrs in a thermostatically controlled oil bath under a nitrogen atmosphere and then reduced to 70°C for another 16 hrs. A sample was removed for NMR analysis. A conversion of 93% GMA and 89% EHMA was measured by ¹H NMR in CDCl₃.

### a) Synthesis of block Bfrom block A

30 minutes before the end of the first synthesis, MAA (4.788g, 55.6mmol, 56.4eq), AIBN (0.077g, 0.5mmol, 0.5eq) and IPA (solvent, 24.503g, 408.7mmol) were added in another flask containing a magnetic stirrer. The mixture was degassed by nitrogen bubbling.

At the end of the first synthesis, the thermostatically controlled oil bath was removed to stop polymerisation. The mixture containing the second monomer was then transferred into the initial two necked flask via a cannula. This flask was heated again at 82°C for 4hrs in the thermostatically controlled oil bath (equipped with a cooling column) under nitrogen atmosphere and then reduced to 70°C for 16 hrs to achieve the preparation of the second block of copolymer. Polymers were precipitated out in diethyl ether and dried in a vacuum oven at 40°C.

A conversion of 82% for the MAA was measured by ¹H NMR in DMSO. The resultant product was determined as (PEHMAₓ-r-PGMA_{y})-b-PMAA_{z} copolymer where x = 51, y = 22 and z = 47.

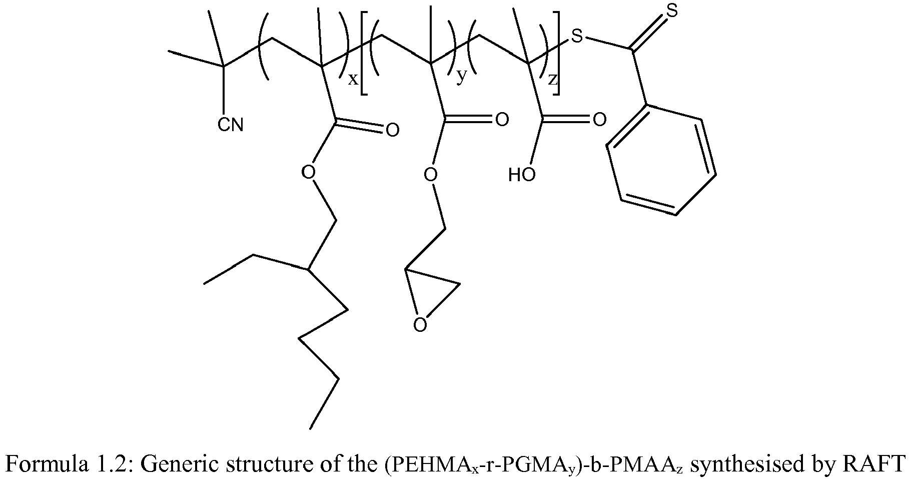

### B) Use of NMP to synthesise copolymers

In this example, according to the protocol described in WO2007/057620-A1, new copolymers structures were produced by NMP polymerization using the NMP agent Blocbuilder®. Whilst the current example prepares the block copolymer using Blocbuilder®, those skilled in the art will appreciate that other NMP agents may be used.

### NMP synthesis of PSₓ-b-(HEMA_{y}-r-PS_{z})

In the first step, the following conditions were used for the synthesis of PS with a targeted polymerisation degree of 55. Styrene (15.00g, 0.14mol) and Blocbuilder® (1.00g, 2.62mmol) were added to a 100ml round bottom flask equipped with a magnetic stirrer. The reaction flask was degassed by nitrogen bubbling for 20 minutes and then heated at 90°C in a thermostatically controlled oil bath under a nitrogen atmosphere. After 78 hrs 40 min of polymerization, a sample was withdrawn and analysed by ¹H NMR (CDCl₃). A conversion of 76.9% was determined by ¹H NMR in CDCl₃, hence the resultant product was PSₓ homopolymer where x = 42.

At the end of this step 15g chloroform was added to solubilise PS. The reactive mixture was precipitated drop by drop in 300ml cold methanol and then filtered on paper. The product was dried down in a vacuum oven.

In a second step, newly synthesised PS (1.00g, 0.23mmol), styrene (0.24g, 2.32mmol), HEMA (2.95g, 22.7mmol) and dimethylformamide (DMF, 4.02g, 0.55mmol) were added to a 50ml round bottom flask equipped with a magnetic stirrer. PS was solubilised in DMF by using a sonic bath (20min). The reaction flask was degassed by nitrogen bubbling for 20 minutes and then heated at 90°C in a thermostatically controlled oil bath under a nitrogen atmosphere. After 18 hrs of polymerization, a sample was withdrawn and analysed by ¹H NMR (DMSO). A conversion of 90.0% for HEMA and 8.0% for styrene was determined by ¹H NMR in DMSO, hence the resultant product was PSₓ-b-(HEMA_{y}-r-PS_{z}) diblock copolymer, where x = 42, y = 90 and z = 8.
At the end of this step, 7ml DMF was added to solubilise the copolymer. The reactive mixture was precipitated drop by drop in 300ml cold ether and then filtered on paper. The product was dried down in a vacuum oven.

Other PSₓ-b-(HEMA_{y}-r-PS_{z}) were prepared with x = 86, y = 57and z = 0 and with x = 74, y = 30 and z = 10. The generic structure of the corresponding PSₓ-b-(HEMA_{y}-r-PS_{z}) copolymers is given below.

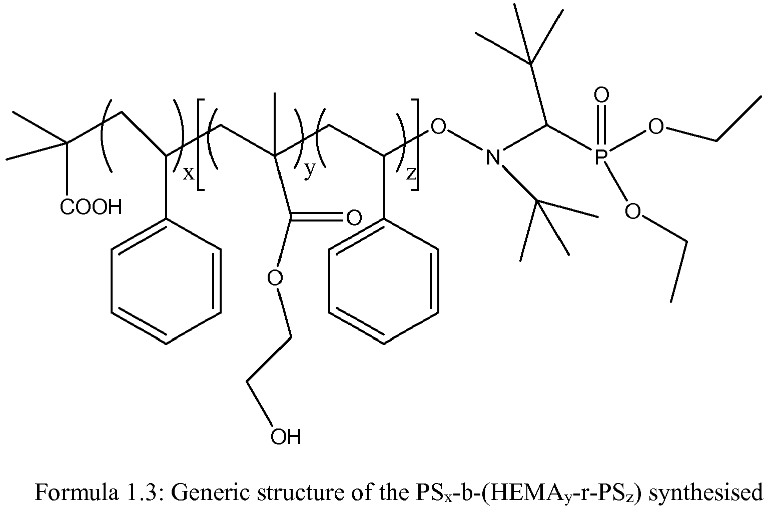

### C) Characterisation

SEC was used to determine the number-average molar mass (Mₙ) and thus demonstrate the increase of molar mass due to the addition of the second block during the polymerisation. SEC was also used to determine the polydispersity index (PDI= M_{w}/Mₙ, where M_{w} is the weight-average molar mass) of the polymers and copolymers, a low PDI being necessary to achieve regular micelles.

The samples were injected in the SEC equipment (2 PL gel 5 Micron Mixed-c columns) and analysis was performed as described below
- The eluent was composed of tetrahydrofuran (THF) for P(EHMAₓ-*b*-MAA_{y}) copolymers and DMF for PSₓ-b-(HEMA_{y}-r-PS_{z}) copolymers (elution flow rate: 1 ml/min, run time: 30 min)
- The calculation (for data analysis) was made with a calibration curve based on poly(methyl methacrylate).
- Before injecting the polymer samples containing methacrylic acid units, a methylation reaction was performed to convert the acid groups into methyl esters, using trimethylsilyldiazomethane as the methylating agent, in order to solubilise the polymers in THF to perform the analysis.
- The samples (20mg) were dissolved in the eluent and then filtered with a 0.2µm PTFE filter into the SEC vials.

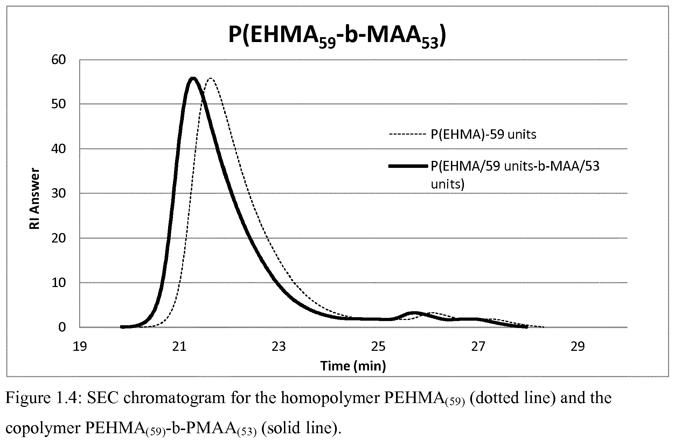

An example of SEC chromatogram is given in Figure 1.4. The SEC chromatogram of the first block of P(EHMA) and the chromatogram of the copolymer P(EHMA-*b*-MAA) are represented. The observed shift of the chromatogram is consistent with an extension of chains between both steps.

**Table 1.1**

| Copolymer | PDI - block 1 | PDI - block 2 |
|---|---|---|
| P(EHMA₅₉-b-MAA₅₃) | 1.68 | 1.85 |
| P(S₄₂-b-[HEMA₉₀-r-S₈]) | 1.31 | 2.03 |
| P(S₈₆-b-HEMA₅₇) | 1.65 | 1.58 |
| P(S₇₄-b-[HEMA₃₀-r-S₁₀]) | 1.43 | 1.84 |
| P(LMA₃₃-b-EHMA₁₀₆) | 1.23 | 1.62 |

Table 1.1: Indication of PDI obtained by SEC for some copolymers described in Table 1.2
-¹H NMR was used to determine the conversion of each polymerisation and the degree of polymerisation (in number: DPₙ) calculated accordingly for each block.
¹H NMR was performed with a 500 MHz apparatus (Bruker), in CDCl₃ for the homopolymer, and in DMSO for the copolymer.

**Table 1.2**

| | Block 1 | | | Block 2 | | |
|---|---|---|---|---|---|---|
| Copolymer | DPn th (units) | Conv. / time | DPn exp x = | DPn th (units) | Conv. / time | DPn exp y = |
| P(EHMAₓ-b-MAA_{y}) ⁽ⁱ⁾ | 60 | 98%, 3h15 | 59 | 60 | 88%, 2h35 | 53 |
| P(Sₓ-b-[HEMA_{y}-r-S_{z}]) ⁽ⁱⁱ⁾ | 80 | 92%, 70h30 | 74 | y: 50 | 60%, 24h | y = 30 |
| | | | | z: 10 | | z = 10 |
| P(Sₓ-b-HEMA_{y}) ⁽ⁱⁱ⁾ | 55 | 76.9%, 78h40 | 42 | y: 100 | 90%, 18h | y = 90 |
| | | | | z: 10 | | z = 8 |
| P(LMAₓ-b-EHMA_{y}) ⁽ⁱⁱⁱ⁾ | 35 | 95.3%, 1h30 | 33 | 106 | 100%, 2h05 | 106 |
| P(EHMAₓ-b-ODMA_{y}) ⁽ⁱⁱⁱ⁾ | 42 | 95.2 % 1h15 | 40 | 70 | 100 %, 19h00 | 70 |

Table 1.2: Synthesis and composition data according to ¹H NMR; EHMA: 2-ethyl hexyl methacrylate; HEMA: 2-hydroxyethyl methacrylate ; MAA: methacrylic acid; S: styrene; LMA: lauryl methacrylate; ODMA: octadecyl methacrylate; Conv.: conversion given in %; DPn th: degree of polymerisation targeted; DPn exp: degree of polymerisation calculated;
i) Synthesis performed using RAFT in IPA
ii) Synthesis performed using NMP in DMF
iii) Synthesis performed using RAFT in toluene

### Example 2: Demonstration of micelle formation

Micellar aggregates can be formed from the copolymers of Example 1. Size distribution measurements using a Malvern Nano Zetasizer were performed on solutions of in apolar solvents such as dodecane, hexane, Exxsol D140, Solvesso 200ND and Isopar V.
1. To demonstrate the formation of micelles in apolar solvent, a solution (10 to 20ml) of copolymer was prepared by dissolving the copolymer powder in THF (Sigma-Aldrich) (1 wt%).
2. When the polymer had dissolved, a second solvent as indicated in Table 2.1 was added drop-by-drop until it reached a large enough quantity that it became the continuous phase. For size distribution measurements, this was when the concentration of copolymer reached ∼0.01 wt%.
3. To ensure that equilibrium was reached the mixture was gently agitated for over 1 hour (mixing with a magnetic stirrer set on low).

To ensure an accurate measurement by the Malvern Nano Zetasizer, concentrations of the copolymer solution were varied so the sample was in the optimum detection range of the instrument for the polymer being examined. The size distribution measurements shown in Table 2.1 shows that the copolymers form micelles, since the minimum diameter measured is 20 nm and if copolymers were present as unimers, the diameter would be less than 5nm. In all cases a clear solution was formed following stage 1. The results in Table 2.1 demonstrate that in each case micelles were formed following stage 3.

Examples of hydrophobic copolymer micelles solutions which were prepared according to the general procedure are described in Table 2.1.

| size distribution following stage 3 (nm) | | | | |
|---|---|---|---|---|
| Dodecane | Hexane | Exxsol D140 | Solvesso 200ND | Isopar V |
| 230 - 240 | 100 - 110 | 95 - 105 | 20 - 80 | 25 - 70 |

Table 2.1: Micelle size measurements of copolymer P(Ethyl Hexyl MA(29)-b-MAA(48)) in apolar liquid media. Measurements collected using a Malvern Nano Zetasizer.

### Example 3 - The preparation and application of a surface treatment according to the present disclosure.

### 3.a Coating a large surface

A polymer or polymeric composition prepared according to the present disclosure may be coated onto a preferred substrate, as described hereafter, by any established coating process, for example, but not limited to a spray process. Generally, the treatment process involves the following steps:
Step (1): Dissolution of the copolymer molecules in a suitable organic solvent under gentle agitation.

The copolymers chosen are usually not of a high molecular weight (the copolymers typically have a range of between 3000 to 100000 g/mol) and such molecules equilibrate rapidly when dissolved in a good solvent.

Solvents suitable for use in the composition of the present disclosure are preferably as previously described.

Step (2): Slow drop-by-drop addition of a second solvent, which is a poor solvent for one of the blocks and a good solvent for the other block, under gentle agitation.

Agitation of the copolymer systems was used during the process of dissolution and mixing, but it was not found to be critical and simply slowly stirring the copolymer system was found to be sufficient. The length of time for agitation depended on the solvent system.

Examples of hydrophobic copolymer micelle solutions which were prepared according to the precedent protocol are described in Table 3.1.

**Table 3.1**

| Mixtures | Copolymer actives | Concentration of actives (wt %) | Step 1: Addition of the good solvent | | Step 2: Addition of the second solvent | |
|---|---|---|---|---|---|---|
| | | | Name | weight (g) | Name | weight (g) |
| 1 | PEHMA₅₉-b-PMAA₅₃ | 0.05 | THF | 0.20 | Ethyl acetate | 19.79 |
| 2 | | 0.4 | THF | 0.20 | Ethyl acetate | 19.79 |
| 3 | | 0.05 | THF | 0.8 | Hexane | 19.12 |
| 4 | | 0.4 | THF | 0.8 | Hexane | 19.12 |
| 5 | | 0.4 | THF | 0.96 | Hexane | 9 |
| 6 | | 0.4 | THF | 0.96 | Isopar IV | 9 |
| 7 | | 0.4 | Toluene | 0.96 | Hexane | 9 |
| 8 | | 0.4 | Toluene | 0.96 | Isopar IV | 9 |
| 10 | PEHMA₂₉-b-PMAA₄₈ | 0.4 | THF | 0.96 | Hexane | 9 |
| 11 | | 0.4 | THF | 0.96 | Isopar IV | 9 |
| 12 | | 0.4 | Toluene | 0.96 | Hexane | 9 |
| 13 | | 0.4 | Toluene | 0.96 | Isopar IV | 9 |
| 14 | PTFEMA₅₉ -b-PMAA₅₁ | 0.4 | Methanol | 0.83 | Hexane | 19.12 |
| 15 | PS₂₂-b-PHEMA₆₀ | 0.05 | DMF | 0.19 | Ethyl acetate | 19.79 |
| 16 | | 0.8 | DMF | 0.72 | Ethyl acetate | 19.12 |
| 17 | | 0.05 | DMF | 0.19 | Hexane | 19.79 |
| 18 | | 0.8 | DMF | 0.72 | Hexane | 19.12 |

Table 3.1: Description of hydrophobic copolymer micelle solutions prepared according to the present disclosure

Step (3): Applying the solution of the copolymer to a desired substrate.

Whilst not wishing to be bound by any particular theory, evidence from the present disclosure implies that adsorption of the copolymer onto the substrate is complete after a few minutes. Methods of exposing the substrate to the solution include any known technique for forming a coating from a solution such as spin coating, dip coating, roller coating, brush coating, curtain flow or spraying, roller coating, wire-bar coating, extrusion coating, air knife coating, curtain coating or slide coating. More preferably, dipping and spraying ensures that every part of the surface has been wetted by the treatment composition.

The treatment can be applied to both interior and exterior surfaces.

Step (4): Drying the treated surface.
Preferably the treated surfaces need to be dried after applying the treatment composition. This can be achieved at room temperature or at higher temperatures, and/or at lower pressure. It should be noted that the drying temperature does not enhance the performance of the coating; rather it shortens the drying time of the treatment. Drying in ambient conditions will only lengthen the drying time.

### 3.b Coating of a discrete object

A copolymer solution was prepared by dissolving the copolymer in a good solvent (toluene / THF) under gentle agitation. Once a homogeneous solution was obtained a second solvent (for example hexane / Isopar V) was added to the mixture using drop-by-drop addition. The final concentration of the copolymer in solution was 0.4 wt%. The second solvent was selected to be a poor solvent or non-solvent for one of the blocks and a good solvent for the other block. The mixture was gently stirred and left for more than 2 hours in order to allow the copolymers to equilibrate into micelles. When the micelle system had reached equilibrium, 1g of TMX (thiamethoxam) air milled crystals were added to the mixture. The sample was then allowed to tumble for at least 2 hours in order to ensure complete mixing and thus allow time for the micelles to coat the individual TMX crystals. Table 3.2 shows possible but not limiting combinations of copolymers and organic solvents which form micelles and can be used to coat crystals.

**PMAA: polymethacrylic acid; PEHMA polyethylhexylmethacrylate**

| **Mixtures** | **Copolymer actives** | **Step 1: Addition of the good solvent** | | **Step 2: Addition of the second solvent** | |
|---|---|---|---|---|---|
| | | **Name** | **Volume (g)** | **Name** | **Volume (g)** |
| 1 | **PEHMA(59)-b-PMAA(53)** | Toluene | 0.96 | Hexane | 9 |
| 2 | **PEHMA(59)-b-PMAA(53)** | Toluene | 0.96 | Isopar V | 9 |
| 3 | **PEHMA(59)-b-PMAA(53)** | THF | 0.96 | Hexane | 9 |
| 4 | **PEHMA(59)-b-PMAA(53)** | THF | 0.96 | Isopar V | 9 |
| 5 | **PEHMA(29)-b-PMAA(48)** | Toluene | 0.96 | Hexane | 9 |
| 6 | **PEHMA(29)-b-PMAA(48)** | Toluene | 0.96 | Isopar V | 9 |
| 7 | **PEHMA(29)-b-PMAA(48)** | THF | 0.96 | Hexane | 9 |
| 8 | **PEHMA(29)-b-PMAA(48)** | THF | 0.96 | Isopar V | 9 |

Table 3.2: Composition of copolymers solutions in apolar hexane and in Isopar V

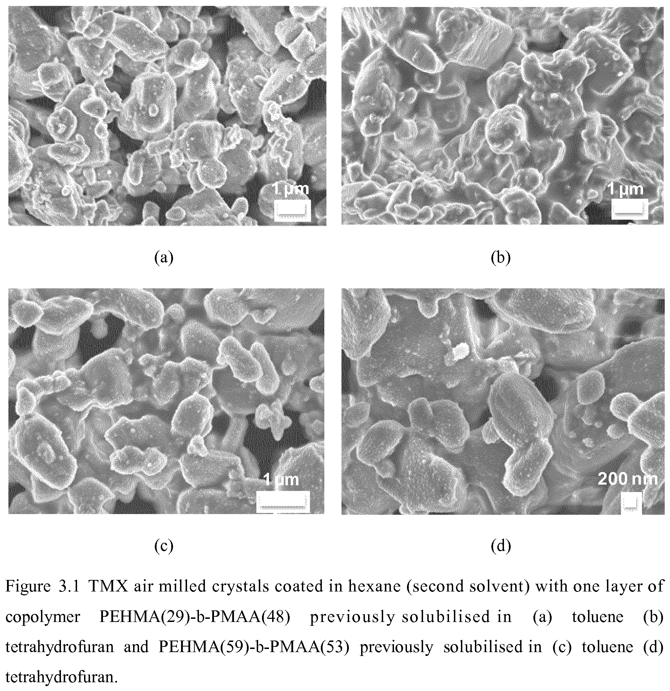

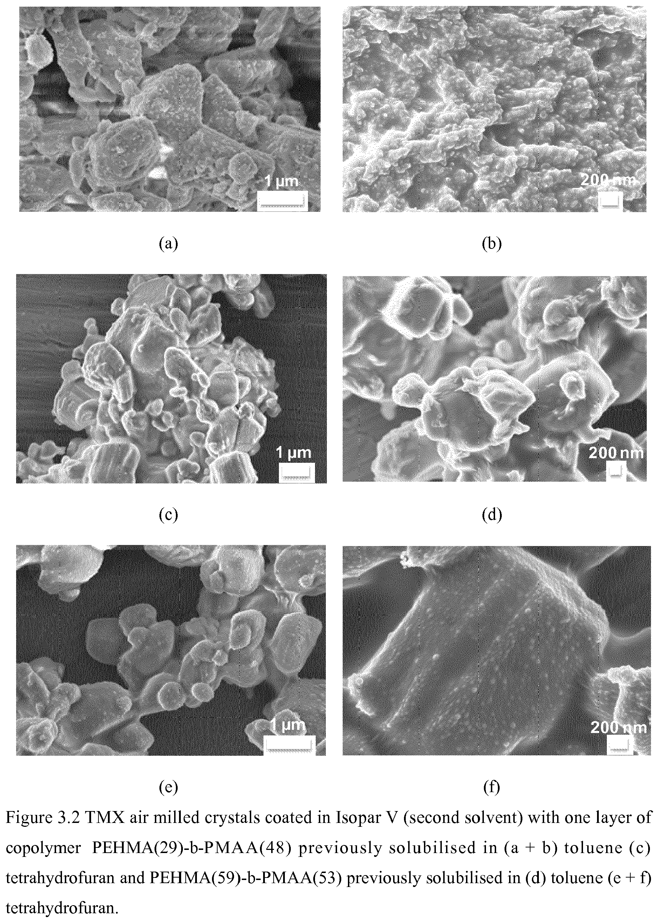

The copolymer solutions in Table 3.2 were used to coat TMX particles using the methodology outlined previously. Figures 3.1 and 3.2 demonstrate that micelles have been deposited from the organic solutions.
Figures 3.1 and 3.2 clearly illustrate the deposition of micelles from a range of organic solvents on all crystal faces, including corners and edges of TMX.

### Example 4: Crosslinking of copolymer micelles

Crosslinking is described as the physical and/or chemical interaction between chains of the AB diblock copolymer. The crosslinking can take place either in the core of the micelles, in the corona of the micelles and/or between the coronas of two contiguous micelles.

In this example, crosslinking of copolymer micelles was used to decrease the solubility of a coated crystalline material in water. Micelles comprising AB di-block copolymers were deposited on the surface of crystals of a crystalline material (for example a pharmaceutical or an agrochemical) in oil based liquid media. Addition of either linear or cyclic diamine molecules to this system led to the modification of the topology of the micellar coating. This also resulted in a decrease in the release rate of the crystalline material in water compared to crystalline material coated with un-crosslinked copolymer micelles.

### Example of crosslinking in oil based system

TMX was coated by using the same protocol as example 3b.

A copolymer solution (10g) was prepared by dissolving (PEHMA₅₁-r-PGMA₂₂)-b-PMAA₄₇ copolymer in a good solvent (THF) under gentle agitation. Once a homogeneous solution was obtained a second solvent (hexane) was added to the mixture using drop-by-drop addition. The final concentration of the copolymer in solution was 0.4 wt%. The second solvent was selected to be a poor solvent or non-solvent for one of the blocks and a good solvent for the other block. The mixture was gently stirred and left for 24 hours in order to allow the copolymers to equilibrate into micelles. When the micelle system had reached equilibrium, 1g of TMX air milled crystals were added to the mixture. The sample was then allowed to tumble for 24 hours in order to ensure complete mixing and thus allow time for the micelles to coat the individual TMX crystals.

Crosslinking was then performed.
1. A diamine compound (see Table 4.1 for mass and molar ratio compared to MAA functions in the copolymer) was added to the solution and tumbled for 24 hours.
2. The mixture was then centrifuged for 2 minutes at 2000 rpm and approximately 9ml of the supernatant liquid was removed. The same quantity of water based TMX saturated stock solution was added, and the mixture tumbled again for 30 minutes.
3. The mixture was then centrifuged for 2 minutes at 2000rpm and 9ml the supernatant liquid was removed.
4. The sample was then dried under vacuum at 50°C for 8 hours thus removing all remaining solvents.

**Table 4.1**

| Cross-linker | Molar ratio of (carboxylic acid + epoxy) functions compared to amine | Mass of crosslinker used (g) |
|---|---|---|
| Control (coated but not crosslinked) | - | |
| Hexamethylene diamine (0.4 wt%) | 1 : 3.8 | 0.0348 |
| Hexamethylene diamine (5 wt%) | 1 : 1.1 | 0.1566 |

Table 4.1: Percentages weight loss of the coated TMX particles

To perform release rate analysis 45-55mg of each sample was accurately weighted into a 60ml powder jar and 50ml of dispersant solution (0.1%w/w Aerosol OTB, 0.5%w/w Morwet D425 in DI water) added at time zero. The samples were then placed on a roller moving at 20rpm. A time point measurement of TMX in solution was made by extracting 3ml of solution and passing it through a 0.45µm filter. The filtrate was then analysed by HPLC to determine the concentration of TMX. The analysis was carried out by High-performance liquid chromatography (HPLC) using an Agilent 1100 (equipped with an auto-injector), a 50 X 3.0 MM ACE 3µM C18 COLUMN FROM ACE, PART NUMBER ACE-111-0503 and mobile phases of (A) Acetonitrile + 0.1% Formic acid and (B) ASTM II Water + 0.1% Formic acid. Analysis was carried out with an injection load of 5 µl and column temperature of 40°C. Data were collected at a range of time points

Total TMX content of the samples was determined by weighing 30-50mg of each dry powder accurately weighted into an aluminium weighting boat. The weighing boat was then placed in a volumetric flask and 50 ml acetonitrile added. The flask was gentle swirled until a colourless solution was formed. This solution was analysed using the HPLC conditions described previously.

Table 4.2 shows the quantity of TMX released after 1 and 4 hours as a percentage of the total TMX concentration as measured by the method described previously.

**Table 4.2**

| time (hours) | Poly(EHMA₅₁-r-GMA₂₂-b-MAA₄₇) no crosslinker | Poly(EHMA₅₁-r-GMA₂₂-b-MAA₄₇) crosslinked (1:1.5 GMA to HMDA ratio) | Poly(EHMA₅₁-r-GMA₁₃-b-MAA₂₁) (1:1.5 GMA to HMDA ratio) |
|---|---|---|---|
| 1 | 79 | 36 | 59 |
| 4 | 84 | 70 | 70 |

### Example 5: Surface characterisation after the application of hydrophobic copolymer micelle solution

The mixture containing the micelles formed from the copolymers PEHMA-b-PMAA in hexane (Table 5.1 - row 1) was used to treat glass microscope slides and painted Q-panels by dip coating, as well as Poly(methyl methacrylate) (PMMA), Polyethylene terephthalate (PET) and Polyvinyl chloride (PVC) sheets by flow coating. The treatment led to an increase of the overall hydrophobicity of the surface as indicated by the contact angle increase of the surface after treatment (Table 5.1)

**Table 5.1**

| | Before treatment | After treatment |
|---|---|---|
| Glass panel (i) | 25 ±1 | 60 ±2 |
| Painted Q-Panel (i) | 69 ±2 | 94.6 ±0.6 |
| PET (ii) | 80.9 ±0.2 | 95.3±0.1 |
| PMMA (ii) | 102.9±0.2 | 106.2±0.1 |
| PVC (ii) | 85.3±0.1 | 93.7±0.2 |

Table 5.1: Contact angle before and after treatment with hydrophobic micelles formed using the block copolymers PEHMA-b-PMAA at 0.4 wt% in hexane
(i) Coating with PEHMA59-b-PMAA53
(ii) Coating with PEHMA59-b-PMAA52

### Example 6: Use of oil-based micelles to coat -actives used in the field of laundry

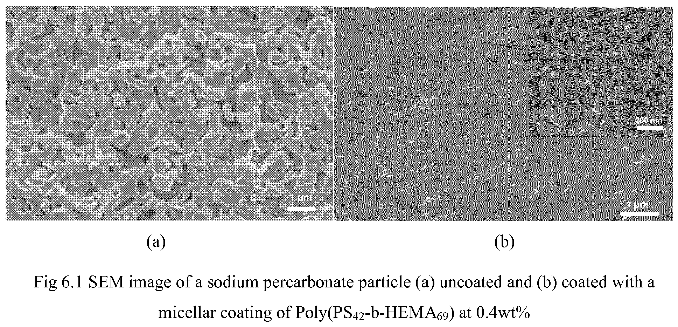

The protocol described in example 3 was used to coat sodium percarbonate with a copolymer micellar solution of Poly(PS₄₂-b-HEMA₆₉) at 0.4 and 5 wt% in DMF/Solvesso 200 - see Figure 6.1.

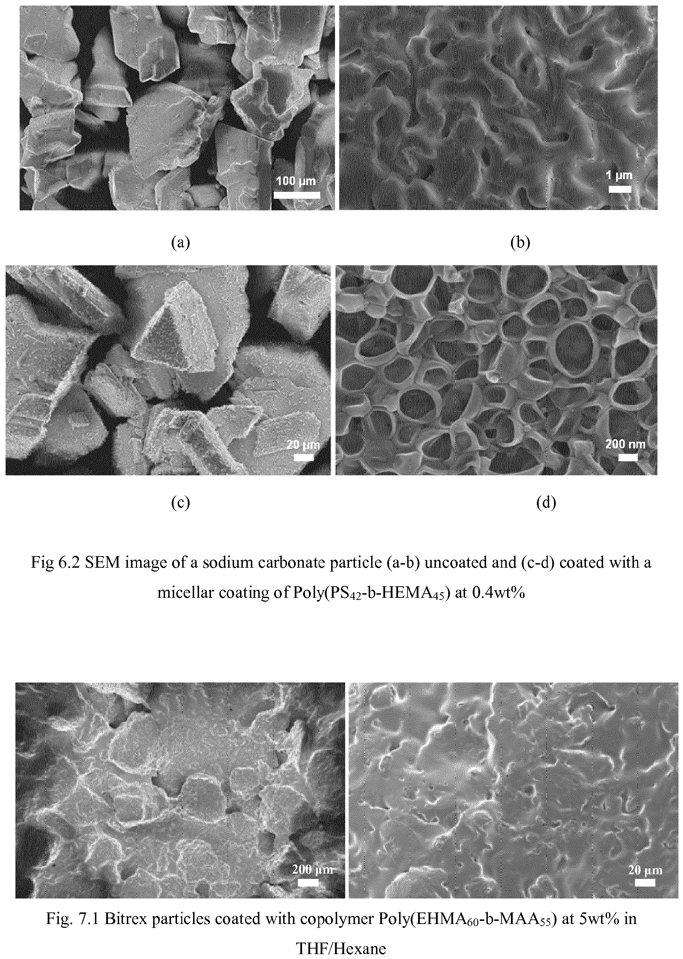

The protocol described in example 3 was used to coat sodium carbonate with a micellar solution of Poly(PS₄₂-b-HEMA₄₅) at 0.4 and 5 wt% in DMF/Solvesso 200 - see Figure 6.2.

### Example 7: Use of oil-based micelles to coat - actives used in the field of taste masking

Bitrex was chosen as it is the bitterest chemical known to man, and has similar physical and chemical characteristics to many pharmaceuticals.

Denatonium benzoate (Bitrex) crystals were coated by adding a copolymer micellar solution of Poly(EHMA₆₀-b-MAA₅₅) at 0.4 and 5 wt% in DMF/Solvesso.

The protocol described in Example 3 was used to coat Bitrex - see Figure 7.1.

### Visual release rate test

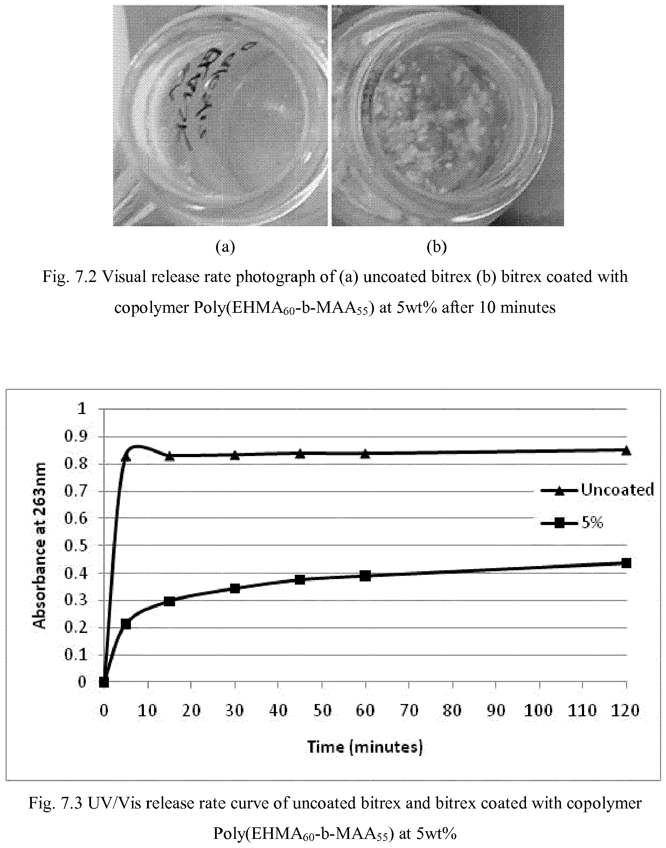

Release rate was monitored visually in order to compare the uncoated Bitrex with the 5 wt% coated Bitrex particles. 0.4mg of sample was agitated in 10ml of water and observed over 8 hours. After 15 minutes the uncoated Bitrex was fully dissolved but after 8 hours the coated particles were still present - see Figure 7.2.

### UV/Vis release rate measurement

100mg in 40ml water of uncoated bitrex and 5 wt% coated Bitrex were shaken for a period of 10 minutes and sampled at various time intervals. 2ml of the mixture was removed at each time interval for analysis.
Total content of the samples was determined by accurately weighing 17.5mg of 5 wt% coated Bitrex particles, sonicated until the coated particles had fully dissolved in 25ml of water and analysed by UV/Vis. A total content measurement of 57.75% was obtained. See Figure 7.3.

| **Time (minutes)** | **% release of Bitrex** | |
|---|---|---|
| | **Uncoated** | **Coated** |
| 5 | 99.6 | 46.5 |
| 15 | 99.8 | 64.9 |

Table 7.1 % release of micelle coated and uncoated Bitrex relative to the total content as determined by UV/vis measurements.

## Claims

1. A process for coating an object with micelles which comprise an AB block copolymer comprising the step of treating the object with an apolar liquid containing the micelles; where the object is an agrochemical organic crystal.

2. A process as claimed in claim 1 where the micelles are crosslinkable and are optionally crosslinked before, during or after treating the object with the apolar liquid containing the micelles.

3. A process as claimed in claim 1 or 2 where the cores of the micelles contain a chemical.

4. A process as claimed in claim 3 where the chemical contained in the cores of the micelles is a photoprotectant.

5. A process as claimed in claim 3 where the chemical contained in the cores of the micelles is a biologically active compound.

6. A process as claimed in claim 3 where the chemical contained in the cores of the micelles is an adjuvant.

7. A process as claimed in any one of the preceding claims where the apolar liquid is selected from alkanes, halogenated solvents, aromatic solvents and combinations thereof.

8. A process as claimed in any one of the preceding claims where the ratio by weight of block copolymer to the apolar liquid is from 1 : 100,000 to 1 : 1.

## Patentansprüche

1. Verfahren zum Beschichten eines Objekts mit ein AB-Blockcopolymer umfassenden Mizellen, bei dem man das Objekt mit einer die Mizellen enthaltenden apolaren Flüssigkeit behandelt, wobei es sich bei dem Objekt um einen agrochemischen organischen Kristall handelt.

2. Verfahren nach Anspruch 1, wobei die Mizellen quervernetzbar sind und gegebenenfalls vor, während oder nach der Behandlung des Objekts mit der die Mizellen enthaltenden apolaren Flüssigkeit quervernetzt werden.

3. Verfahren nach Anspruch 1 oder 2, wobei die Kerne der Mizellen eine Chemikalie enthalten.

4. Verfahren nach Anspruch 3, wobei es sich bei der in den Kernen der Mizellen enthaltenen Chemikalie um ein Lichtschutzmittel handelt.

5. Verfahren nach Anspruch 3, wobei es sich bei der in den Kernen der Mizellen enthaltenen Chemikalie um eine biologisch wirksame Verbindung handelt.

6. Verfahren nach Anspruch 3, wobei es sich bei der in den Kernen der Mizellen enthaltenen Chemikalie um ein Adjuvans handelt.

7. Verfahren nach einem der vorhergehenden Ansprüche, wobei die apolare Flüssigkeit aus Alkanen, halogenierten Lösungsmitteln, aromatischen Lösungsmitteln und Kombinationen davon ausgewählt ist.

8. Verfahren nach einem der vorhergehenden Ansprüche, wobei das Gewichtsverhältnis von Blockcopolymer zur apolaren Flüssigkeit 1 : 100.000 bis 1 : 1 beträgt.

## Revendications

1. Procédé de revêtement d'un objet avec des micelles qui comprennent un copolymère séquencé AB comprenant l'étape de traitement de l'objet avec un liquide apolaire contenant les micelles ; où l'objet est un cristal organique agrochimique.

2. Procédé selon la revendication 1, dans lequel les micelles sont réticulables et sont facultativement réticulées avant, pendant ou après le traitement de l'objet avec le liquide apolaire contenant les micelles.

3. Procédé selon la revendication 1 ou 2 dans lequel les noyaux des micelles contiennent une substance chimique.

4. Procédé selon la revendication 3 dans lequel la substance chimique contenue dans les noyaux des micelles est un photoprotecteur.

5. Procédé selon la revendication 3 dans lequel la substance chimique contenue dans les noyaux des micelles est un composé biologiquement actif.

6. Procédé selon la revendication 3 dans lequel la substance chimique contenue dans les noyaux des micelles est un adjuvant.

7. Procédé selon l'une quelconque des revendications précédentes, dans lequel le liquide apolaire est choisi parmi des alcanes, des solvants halogénés, des solvants aromatiques et des combinaisons de ceux-ci.

8. Procédé selon l'une quelconque des revendications précédentes, dans lequel le rapport en poids du copolymère séquencé au liquide apolaire est de 1 : 100 000 à 1 : 1.
